(19) Europäisches Patentamt
European Patent Office
Office européen des brevets

(11) EP 0 759 066 B1

(12) **EUROPÄISCHE PATENTSCHRIFT**

(45) Veröffentlichungstag und Bekanntmachung des Hinweises auf die Patenterteilung:
**27.06.2001 Patentblatt 2001/26**

(21) Anmeldenummer: **95919390.5**

(22) Anmeldetag: **29.04.1995**

(51) Int Cl.[7]: **C12N 1/20**, C12N 9/80, C12P 21/00, C12P 41/00, C12P 13/02 // (C12N1/20, C12R1:64)

(86) Internationale Anmeldenummer:
**PCT/EP95/01689**

(87) Internationale Veröffentlichungsnummer:
**WO 95/30740 (16.11.1995 Gazette 1995/49)**

(54) **VERFAHREN ZUR GEWINNUNG VON PEPTIDAMIDASE ENTHALTENDEN MIKROORGANISMEN, DAMIT GEWONNENE MIKROORGANISMEN, DARIN ENTHALTENE PEPTIDAMIDASEN UND DEREN VERWENDUNG**

PROCESS FOR OBTAINING MICRO-ORGANISMS CONTAINING PEPTIDE AMIDASE, MICRO-ORGANISMS OBTAINED THEREWITH, PEPTIDE AMIDASES CONTAINED IN THEM AND USE THEREOF

PROCEDE POUR L'OBTENTION DE MICRO-ORGANISMES CONTENANT UNE PEPTIDE-AMIDASE, MICRO-ORGANISMES AINSI OBTENUS, PEPTIDE-AMIDASES CONTENUES DANS CEUX-CI ET LEUR UTILISATION

(84) Benannte Vertragsstaaten:
**CH DE FR GB IT LI NL**

(30) Priorität: **09.05.1994 DE 4415971**

(43) Veröffentlichungstag der Anmeldung:
**26.02.1997 Patentblatt 1997/09**

(73) Patentinhaber: **Degussa AG**
**40474 Düsseldorf (DE)**

(72) Erfinder:
- **STELKES-RITTER, Ursula**
  **D-52382 Niederzier (DE)**
- **KULA, Maria-Regina**
  **D-52382 Hambach-Niederzier (DE)**
- **WYZGOL, Klaudia**
  **D-40239 Düsseldorf (DE)**
- **BOMMARIUS, Andreas**
  **D-60323 Frankfurt (DE)**
- **SCHWARM, Michael**
  **D-63755 Alzenau (DE)**
- **DRAUZ, Karlheinz**
  **D-63579 Freigericht (DE)**

(56) Entgegenhaltungen:
**EP-A- 0 501 292**
**WO-A-89/01525**
**DE-C- 4 014 564**
**FR-A- 2 586 702**

EP 0 759 066 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die Erfindung bezieht sich auf ein Verfahren zur Gewinnung von Peptidamidase enthaltenden Mikroorganismen, damit gewonnene Mikroorganismen, darin enthaltene Peptidamidasen und deren Verwendung.

Insbesondere betrifft die Erfindung ein Screening-Verfahren für Peptidamidaseaktivität zeigende Mikroorganismen gemäß Oberbegriff des Anspruchs 1; nach diesem Verfahren erhaltene und hinterlegte Mikroorganismen gemäß den Ansprüchen 2 - 4; aus den Mikroorganismen isolierbare Peptidamidasen gemäß Ansprüchen 5 - 7 sowie deren Verwendung.

Zum Stand der Technik werden die Druckschriften

(1) DE-OS 36 29 242 und FR-A-2 586 702,
(2) K. Breddam, Carlsberg Res. Commun. 49 (1984) 535 - 554,
(3) DE-PS 40 14 564 und
(4) Y. Nishida et al., Enzyme Microb. Technol., 6 (1984), 85 - 90

genannt.

Eine Peptidamidase ist ein Enzym, das die selektive Hydrolyse einer C-terminalen Amidfunktion in einem Peptidamid katalysiert, d. h. folgende Umsetzung beschleunigt:

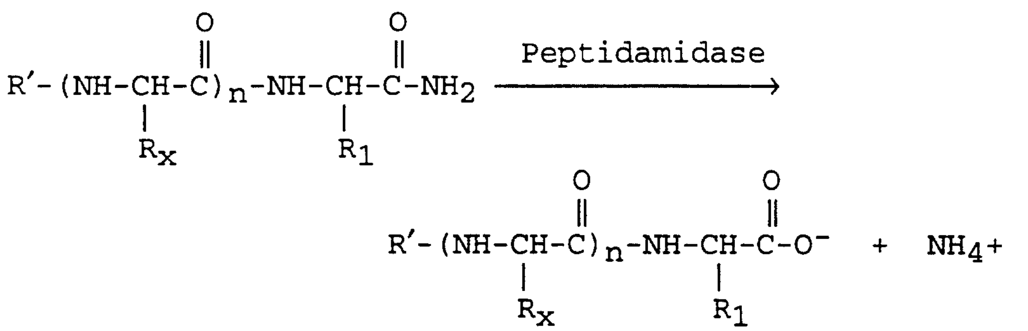


(I)

Hierin bedeuten R' für n = 0 eine Schutzgruppe und für n > 0 eine beliebige Aminosäure, eine Schutzgruppe oder H; n steht für Null oder eine beliebige ganze Zahl, $R_x$ sind die Seitenketten der Aminosäuren für n > 0, während $R_1$ die Seitenkette der C-terminalen Aminosäure bedeutet.

Die selektive Abspaltung der C-terminalen Aminogruppe von Peptidamiden ist durch eine chemische Umsetzung i. allg. schwer zu erreichen, da die Peptidbindung ebenfalls einem hydrolytischen Angriff unterliegt. Daraus ergeben sich schwer zu trennende Gemische und geringe Ausbeuten.

Aus (1) sind Amidasen für eine enzymatische Abspaltung der Säureamidgruppe bekannt, die jedoch aufgrund ihrer α-Aminosäureamidaseaktivität nur zur Herstellung von L-Aminosäuren aus α-ungeschützten D,L-Aminosäureamiden eingesetzt werden können, Peptidamide werden nicht akzeptiert.

Aus (4) ist die kontinuierliche Herstellung von N-Ac-L-Met aus N-Ac-D,L-methioninamid in einem enzymatischen Verfahren unter Verwendung von Erwinia carotovera bekannt.

Erwinia carotovera enthält zwar eine Amidaseaktivität, diese ist jedoch ausschließlich auf Amide des Methionins beschränkt. Damit handelt es sich bei dem Enzym aus Erwinia carotovera nur um einen "Aminosäure-Amidabspalter" und nicht um eine Peptidamidase. Ferner kann das Enzym aus Erwinia carotovera offenbar nur N-acetylierte Aminosäureamide umsetzen, wobei die Ac-Schutzgruppe nachteilig nur schwer oder nicht wieder abspaltbar ist.

Andererseits kennt man Peptidasen, welche die hydrolytische Spaltung der Peptidbindungen katalysieren und von denen lediglich bekannt ist, daß sie eine gewisse Nebenaktivität zur Abspaltung der C-terminalen Amidschutzgruppe besitzen. Ein Beispiel dafür ist die Carboxypeptidase Y, insbesondere in chemisch modifizierter Form (siehe (2)).

Alle diese Verfahren haben also gravierende Nachteile.

Stand der Technik ist gemäß (3) auch eine Peptidamidase, die aus dem Flavedo von Citrusfrüchten, insbesondere von Orangen, isoliert werden kann. Die beschriebene Peptidamidase greift die Peptidbindung nicht an und katalysiert die Abspaltung der freien Aminogruppe von Peptidamiden. Die aus (3) bekannte Peptidamidase kennzeichnet sich durch folgende Parameter:

- Abspaltung der C-terminalen Aminogruppe von Peptidamiden und N-terminal geschützten Aminosäureamiden;

- keine Spaltung von Peptidbindungen;
- optimaler pH-Wert bei 7,5 ± 1,5;
- gute Stabilität im pH-Bereich zwischen pH 6,0 und pH 9,0;
- die optimale Temperatur beträgt 30 °C bei einem pH-Wert von 7,5;
- schwache Inhibierung durch Hemmstoffe von Serinproteasen, insbesondere Phenylmethansulfonylfluorid;
- das Molekulargewicht beträgt 23 000 ± 3 000;
- Aggregatbildung wird gelegentlich beobachtet;
- der isoelektrische Punkt liegt bei pH 9,5;
- das Enzym akzeptiert keine D-Aminosäurereste in C-terminaler Position, wobei die Hydrolysegeschwindigkeit deutlich geringer ist als bei L-Aminosäureresten.

Das isolierte Enzym ist aus Flavedo allerdings nur in geringen Mengen und saisonabhängig zu gewinnen. Auch gelang es in weiterführenden Arbeiten trotz einer ca. 500fachen Anreicherung nicht, das Protein in homogener Form darzustellen, so daß molekular-genetische Arbeiten zur Verbesserung der Enzymproduktion aus Mangel an Daten nicht aufgenommen werden konnten.

Hiermit wird jedoch auch der in (3) gegebene Hinweis gegenstandslos, daß sich eine mikrobielle Produktion des Enzyms durch gentechnologische Manipulation in bekannter Weise erreichen ließ. Gerade die Schwierigkeiten bei der Darstellung der homogenen Form lassen gentechnologische Manipulationen nicht zu.

Angesichts der mit dem Stand der Technik verbundenen Probleme ist es daher Aufgabe der Erfindung, ein Verfahren zur Isolierung von Peptidamidase enthaltenden Mikroorganismen zur Verfügung zu stellen, das eine schnelle Auswahl geeigneter Stämme ermöglicht. Aufgabe ist auch eine stabile Peptidamidase, die bei gleich hoher Selektivität der hydrolytischen Abspaltung der freien Aminogruppe am C-terminalen Ende von Peptidamiden, besser verfügbar ist als die bekannte Peptidamidase aus Flavedo.

Gelöst werden diese und weitere nicht im einzelnen aufgeführte Aufgaben durch ein Verfahren mit den Merkmalen des kennzeichnenden Teils des Anspruchs 1.

Mikroorganismen sind praktisch zu jeder Jahreszeit in beliebiger Menge zu produzieren. Daher werden in einem limitierten Screening Sammlungsstämme und isolierte Stämme, die Amidstickstoff als Stickstoffquelle mobilisieren können, auf Peptidamidaseaktivität untersucht. Als Testsubstrat wurde Z-Gly-Tyr-$NH_2$ eingesetzt und das erwartete Hydrolyseprodukt Z-Gly-Tyr-OH mittels HPLC bestimmt.

Dadurch, daß man in einem "Doppel-Screening" Mikroorganismen enthaltende Proben zuerst in einem Nährmedium, das Amidstickstoff als Stickstoffquelle aufweist, bebrütet und anschließend entstandene Kolonien auf ein Nährmedium überimpft, das N-Acetyl-D,L-Methioninamid enthält, bebrütet und die Mikroorganismen auswählt, die in beiden Nährmedien wachsen, gelingt es in einer ungewöhnlich guten Erfolgsquote Stämme aufzufinden, die sowohl relativ stabil als auch selektiv und aktiv sind.

Für das Screening nach Mikroorganismen, die Amidstickstoff verwerten können, wurden Bodenproben in isotonischer Kochsalzlösung 4 - 6 Stunden aufgeschlämmt, wobei die Bodenproben willkürlichen Ursprungs waren, u. a. aus Gartenerde, Waldboden, Lehm- oder Sandboden stammten. Die Feststoffe wurden bei 2000 Upm durch Zentrifugation abgetrennt. Der Überstand wurde auf Agarplatten ausgestrichen bzw. zum Animpfen von Flüssigmedien in Erlenmeyerkolben genutzt. Die Platten wurden für 3 - 7 Tage bei 30 °C inkubiert, die Erlenmeyerkolben bei der gleichen Temperatur bei 120 Upm geschüttelt. Anschließend wurden von den Platten Einzelkulturen isoliert und durch mehrmaliges Ausstreichen in Reinkultur gebracht. Aus den bebrüteten Erlenmeyerkolben wurden nach dieser Zeit Aliquots entnommen und damit frisches Medium angeimpft. Dieser Vorgang wurde bis zu fünfmal wiederholt, bevor Proben der Kulturflüssigkeit nach geeigneter Verdünnung auf Platten ausgestrichen wurden. Das Nährmedium sowohl für das feste als auch für das flüssige Medium hatte folgende Zusammensetzung:

| | |
|---|---|
| $K_2HPO_4$ | 2.50 g/l |

(fortgesetzt)

| | |
|---|---|
| $KH_2PO_4$ | 1.95 g/l |
| NaCl | 1.00 g/l |
| $CaCl_2*2H_2O$ | 0.05 g/l |
| $MgSO_4*7H_2O$ | 0.30 g/l |
| Hefeextrakt | 0.50 g/l |
| DL-Carnitinamid | 5.00 g/l |
| Spurensalzlösung | 0.80 ml/l |
| Vitaminlösung | 2.5 ml/l |
| (Agar für Festmedien | 18.0 g/l) |
| pH 7.2 | |

$CaCl_2*2H_2O$, $MgSO_4*7H_2O$ sowie DL-Carnitinamid und die Vitaminlösung (s. u.) wurden sterilfiltriert dem autoklavierten, abgekühlten Medium zugesetzt. Die Spurensalzlösung setzte sich wie folgt zusammen:

| | |
|---|---|
| $H_3BO_3$ | 75.0 mg |
| $MnCl_2*4H_2O$ | 50.0 mg |
| $ZnCl_2$ | 187.0 mg |
| $CuSO_4*5H_2O$ | 50.0 mg |
| $FeCl_3*6H_2O$ | 625.0 mg |
| $(NH_4)_8Mo_7O_{24}*4H_2O$ | 25.0 mg |
| $CoSO_4*7H_2O$ | 37.5 mg |
| $H_2O$ demin. | ad. 0.2 l |

Auf diesem Medium gewachsene und vereinzelte Mikroorganismen wurden für das Screening nach Organismen mit Peptidamidaseaktivität eingesetzt.

Für das Screening nach Mikroorganismen mit Peptidamidaseaktivität wurde ein Teil der oben gewonnenen Organismen auf Agarnährboden ausgestrichen und für 2 Tage bei 30 °C inkubiert. Zur Gewinnung höherer Zellmassen wurden die Kulturen in 100 ml Erlenmeyerkolben mit 20 ml Medium angereichert. Die Inkubation erfolgte bei 30 °C für 2 Tage bei 120 Upm. Das hierfür benutzte Nährmedium hatte folgende Zusammensetzung:

| | |
|---|---|
| $KH_2PO_4$ | 0.50 g/l |
| $K_2HPO_4$ | 2.00 g/l |
| NaCl | 1.00 g/l |
| $CaCl_2*2\ H_2O$ | 0.05 g/l |
| $MgSO_4*7H_2O$ | 0.10 g/l |
| Glucose | 5.00 g/l |
| DL-Carnitinamid | 1.00 g/l |
| N-Ac-DL-Met-$NH_2$ | 2.00 g/l |
| Hefeextrakt | 0.50 g/l |
| Vitaminlösung | 2.50 ml/l |
| Spurensalzlösung | 0.80 ml/l |
| (Agar für Festmedien | 18.00 g/l |
| pH 7.3 | |

Dabei wurde N-Ac-D,L-Met-$NH_2$ als Induktor eingesetzt. Glucose als Kohlenstoffquelle sollte vermeiden, daß der Induktor vom N-Terminus her angegriffen wird. Amide, Glucose, $CaCl_2$ $*2H_2O$, $MgSO_4*7H_2O$ und die Vitaminlösung wurden sterilfiltriert dem autoklavierten, abgekühlten Nährmedium zugesetzt (Zusammensetzung der Spurensalzlösung und der Vitaminlösung siehe unten).

Die Zellen wurden zentrifugiert, mit 50 mM Tris/HCl, pH 7.5 gewaschen und in eben diesem Puffer aufgenommen (20 - 40 %ige Zellsuspension). Der Zellaufschluß erfolgte durch Naßvermahlung mit Glasperlen von 0.3 mm im Durchmesser.

Die hieraus gewonnenen Rohextrakte wurden auf ihre Fähigkeit zur Hydrolyse von Z-Gly-Tyr-$NH_2$ hin unter-

sucht.

Von 45 auf Desamidierung hin untersuchten Stämmen zeigten 6 die Fähigkeit zur Umsetzung von Z-Gly-Tyr-$NH_2$ zu Z-Gly-Tyr-OH. In Tabelle 1 werden ausgewählte Daten aus dem Screening für Peptidamidasen dargestellt.

Tabelle 1

| Stamm Nr. | $OD_{660}$ * | Proteingehalt [mg BSAeq/ml] | Spez. Aktivität [mU/mgBSAeq] |
|---|---|---|---|
| 4 | 0.98 | 1.93 | 4.84 |
| 11 | 1.22 | 5.30 | 9.70 |
| 18 | 1.13 | 5.96 | 2.97 |
| 21 | 0.87 | 7.90 | 5.22 |
| 22 | 1.04 | 9.40 | 7.23 |
| 42 | 1.58 | 3.46 | 2.82 |

* $OD_{660}$ = Optische Dichte bei einer Wellenlänge von 660 nm.

Die isolierten Stämme 4, 11, 18, 21, 22, 42 wurden bei der Deutschen Sammlung für Mikroorganismen DSM auf den Namen der Anmelderin hinterlegt.

| Stamm Nr. | DSM Nr. | Hinterlegungsdatum |
|---|---|---|
| 4 | 9182 | 02.05.1994 |
| 11 | 9181 | 02.05.1994 |
| 18 | 9183 | 02.05.1994 |
| 21 | 9184 | 02.05.1994 |
| 22 | 9185 | 02.05.1994 |
| 42 | 9186 | 02.05.1994 |

Von den hinterlegten Stämmen wurde Stamm 11 für weitere Untersuchungen ausgewählt. Von der DSM wurde Stamm 11 als Stenotrophomonas maltophilia (Xanthomonas maltophilia) bestimmt. Die Stämme 4, 18, 21, 22 und 42 wurden ebenfalls taxiert, mit dem Ergebnis, daß die Stämme 4, 18, 21 und 22 ebenfalls als Stenotrophomonas maltophilia (Xanthomonas maltophilia), der Stamm 42 als Ochrobactrum anthropi identifiziert wurden.

Gegenstand der Erfindung sind auch aus den gescreenten Mikroorganismen erhältliche Peptidamidasen.

Die Peptidamidase wird intrazellulär gebildet und kann nach dem Fachmann geläufigen Verfahren aus den Zellen isoliert und gereinigt werden.

Die mikrobielle Peptidamidase kennzeichnet sich durch folgende Parameter

- Abspaltung der C-terminalen Aminogruppe von Peptidamiden und N-terminal geschützten Aminosäureamiden;
- keine Spaltung von Peptidbindungen;
- optimaler pH-Wert bei 6,0 ± 0,5;
- gute Stabilität im pH-Bereich zwischen pH 7 und pH 8;
- die optimale Temperatur beträgt 35 - 40 °C bei einem pH-Wert von 7,5;
- Inhibierung von Serinresten durch Hemmstoffe, wie Phenylmethansulfonylfluorid, sowie insbesondere 4-(2-Aminoethylbenzylsulfonylfluorid) (Pefabloc);
- das Molekulargewicht beträgt etwa 38.000 Dalton (bestimmt durch Gelfiltration);
- der isoelektrische Punkt liegt bei pH 5,8.

Die Erfindung umfaßt ebenfalls die isozymen Formen der erfindungsgemäßen mikrobiellen Peptidamidase. Unter isozymen Formen werden dabei die Enzyme in anderen Mikroorganismen verstanden, die dieselbe Reaktion katalysieren wie die Peptidamidase aus Xanthomonas maltophilia.

In Tabelle 2 werden einige Daten zur Aufreinigung der Peptidamidase aus Xanthomonas maltophilia angegeben.

Tabelle 2

| | Spez. Aktivität [mU/mg BSAeq] | Aufreinigung [-] | Ausbeute [%] |
|---|---|---|---|
| Rohextrakt | 4 | 1 | 100 |
| Q-Sepharose FF + Ultrafiltration | 224 | 56 | 97 |
| Superdex G 75 + Ultrafiltration | 1034 | 256 | 75 |
| IEF an der Mono P | 2133 | 533 | 63 |

Die gereinigte Peptidamidase aus Xanthomonas maltophilia hat insbesondere folgende charakteristische Eigenschaften:

- Aufreinigung von > 500 bei einer Ausbeute von > 60 %
- Molekulargewicht von 38000 Da (Gelfiltration)
- isoelektrischer Punkt liegt bei pH 5.8
- Temperaturoptimum zwischen 37 - 45 °C
- pH Optimun zwischen 5 - 6.5
- bei 20, 30 und 37 °C über 3 Tage temperaturstabil
- bei pH 7 - 8 ist das Enzym bei 30 °C über 7 Tage stabil
- bei Zusatz von 20 % DMF zeigt die Peptidamidase nach 24 h eine Restaktivität von 32 % bei einem pH-Wert von 7.5
- Serinrest entscheidend für Enzymaktivität.

Die N-terminale Anfangssequenz der Peptidamidase aus Xanthomonas maltophilia ist:

| | |
|---|---|
| AS 1 | X |
| AS 2 | Arg |
| AS 3 | Asn |
| AS 4 | Val |
| AS 5 | Pro |
| AS 6 | Phe |
| AS 7 | Pro |
| AS 8 | Tyr |
| AS 9 | Ala |
| AS 10 | Glu |
| AS 11 | Thr |
| AS 12 | Asp |
| AS 13 | Val |
| AS 14 | Ala |
| AS 15 | Asp |
| AS 16 | Leu |
| AS 17 | Gln |

Die erste Aminosäure konnte nicht bestimmt werden. In dem Programm Genepro 5.0 der Datenbank PIR Version 30 konnte keine vergleichbare Sequenz ermittelt werden.

In der folgenden Tabelle 3 sind die Eigenschaften der bekannten pflanzlichen Peptidamidase aus Flavedo von Orangen (PAF) und der erfindungsgemäßen mikrobiellen Peptidamidase aus Xanthomonas (PAX) verglichen.

Die erfindungsgemäße Peptidamidase zeigt danach weder Peptidaseaktivität noch Aminosäureamidaseaktivität.

Tabelle 3

| | **PAF** | **PAX** |
|---|---|---|
| Spez. Aktivität [U/mg BSAeq] | 100.1 | 2.13 |
| Aminosäureamidaseaktivität | 0 | 0 |
| Peptidaseaktivität | 0 | 0 |
| Molmasse (Gelfiltration) | 23000 | 38000 |
| pH-Optimum | 6.5 - 8.7 | 5 - 6.5 |
| Temperaturoptimum | 30 - 35 °C | 37 - 45 °C |
| Isoelektrischer Punkt | 9.5 | 5.8 |
| N-terminale Sequenz | nicht bekannt | |

Die zur Erfindung gehörigen mikrobiellen Peptidamidasen eignen sich sehr vorteilhaft zur Katalyse einer ganzen Reihe von Reaktionen.

So lassen sich die erfindungsgemäßen mikrobiellen Peptidamidasen erfolgreich zur Herstellung von Peptiden und N-terminal geschützten Aminosäuren der allgemeinen Formel II

$$\begin{array}{l} R'\text{-NH-CH-COOH} \\ \qquad\quad | \\ \qquad\quad R_1 \end{array} \qquad\qquad (II)$$

in der R' eine Schutzgruppe oder ein beliebiger peptidisch oder isopeptidisch gebundener Aminosäure- oder Peptidrest ist und $R_1$ Wasserstoff oder eine beliebige Seitenkette bedeutet, unter enzymatischer Abspaltung einer C-terminalen Aminogruppe von einem Peptidamid oder einem N-terminal geschützten Aminosäureamid einsetzen.

In bevorzugter Verfahrensvariante wird diese enzymatische Reaktion kontinuierlich durchführt.

Weiterhin ist es bevorzugt, daß man die Desamidierung als Verfahrensschritt einer gekoppelten Umsetzung mit einem Enzymsystem durchführt, das Proteasen, Peptidasen, Esterasen und/oder Lipasen umfaßt. Hierbei ist besonders die Selektivität der erfindungsgemäßen Peptidamidasen von Vorteil.

Die mikrobiellen Peptidamidasen lassen sich erfindungsgemäß auch in Verfahren zur Herstellung von Peptiden der oben erwähnten Art durch enzymatische Umsetzung von ggf. N-geschützten Aminosäurealkylestern bzw. ggf. N-geschützten Peptidalkylestern mit Aminosäureamiden in wäßriger Phase oder wäßrig-organischem Milieu verwenden. Hierbei verläuft die Reaktion in Gegenwart eines die peptidische Verknüpfung herbeiführenden Enzyms und unter enzymatischer Abspaltung der Amidschutzgruppe, wobei man die Synthese kontinuierlich ablaufen läßt und das Peptidamid mittels der Peptidamidase enzymatisch zum Peptid hydrolysiert und schließlich das Peptid aufgrund seiner Ladung vom Reaktionsgemisch abtrennt und das Aminosäureamid zurückführt.

Weiterhin kann die mikrobielle Peptidamidase der Erfindung auch zur Racematspaltung von N-geschützten Aminosäureamiden verwendet werden, wobei ein racemisches Gemisch von N-geschützten Aminosäureamiden mit der Peptidamidase inkubiert wird und bis zum vollständigen Umsatz des N-geschützten L-Aminosäureamids reagiert wird und nachfolgend die N-geschützte L-Aminosäure vom N-geschützten D-Aminosäureamid aufgrund der Ladungsunterschiede getrennt wird. Ferner lassen sich erfindungsgemäß auch D-Aminosäuren herstellen. So kann beispielsweise im Rahmen der Erfindung durch Verwendung der erfindungsgemäßen mikrobiellen Peptidamidase selektiv das $N_\alpha$-geschützte L-Aminosäureamid enzymatisch hydrolysiert werden, das $N_\alpha$-geschützte D-Aminosäureamid abgetrennt und mittels Säurehydrolyse in die freie D-Aminosäure überführt werden.

Schließlich lassen sich die erfindungsgemäßen mikrobiellen Peptidamidasen mit großem Vorteil auch zur Gewinnung nichtproteinogener D-Aminosäuren einsetzen, wobei man bevorzugt sterisch anspruchsvolle, N-geschützte racemische Aminosäureamide wie N-Acetyl-Neopentylglycinamid, N-Acetyl-Naphthylalaninamid, N-Acetylphenylglycinamid oder ähnliche Derivate einsetzt. Die N-Acetyl-L-Aminosäureamide werden enzymatisch hydrolysiert, die N-Acetyl-D-Aminosäureamide durch Chromatographie aus dem Reaktionsgemisch abtrennt und schließlich mittels Säurehydrolyse in die freien D-Aminosäuren überführt.

Zu den durch Verwendung der erfindungsgemäßen mikrobiellen Peptidamidase aus den bevorzugten race-

mischen $N_\alpha$-geschützten Aminosäureamiden erhältlichen $N_\alpha$-geschützten D-Aminosäureamiden gehören z. B.: $N_\alpha$-Formyl-D-methioninamid, $N_\alpha$-Methylaminocarbonyl-D-methioninamid, $N_\alpha$-Methoxycarbonyl-D-methioninamid, $N_\alpha$-Ethoxycarbonyl-D-methioninamid, $N_\alpha$-Benzyloxycarbonyl-D-methioninamid, $N_\alpha$-Acetyl-D-neopentylglycinamid, $N_\alpha$-Benzyloxycarbonyl-D-neopentylglycinamid.

Nachfolgend wird die Erfindung eingehender anhand von Beispielen erläutert. Weitere Ausführungsformen und Besonderheiten ergeben sich insbesondere auch aus den beigefügten Figuren, auf die in der Beschreibung Bezug genommen wird.

In den Figuren zeigen:

Fig. 1: Die Abhängigkeit der Umsatzrate [%] vom pH-Wert für aus Xanthomonas maltophilia gewonnene Peptidamidase

Fig. 2: Eine Auftragung der relativen Aktivität gegen die Zeit für Peptidamidase aus Xanthomonas maltophilia in verschiedenen Puffern bei 30 °C

Fig. 3: Auftragung der Umsatzrate in Abhängigkeit von der Temperatur für Peptidamidase aus Xanthomonas maltophilia

Fig. 4: Temperaturstabilität der Peptidamidase aus Xanthomonas maltophilia

Fig. 5: Kinetische Bestimmung des Umsatzes von Z-Gly-Tyr-$NH_2$ mit der Peptidamidase aus Xanthomonas maltophilia

Fig. 6: Racematspaltung von Z-D,L-Ala-$NH_2$ mit der Peptidamidase aus Xanthomonas maltophilia

Fig. 7: Enantioselektive Desamidierung von N-Ac-D,L-Met-$NH_2$ mit der Peptidamidase aus Xanthomonas maltophilia

Fig. 8: Lösungsmitteleinfluß auf die Enzymaktivität der Peptidamidase aus Xanthomonas maltophilia

Fig. 9: Lösungsmittelstabilität der Peptidamidase aus Xanthomonas maltophilia

Beispiel 1

Produktion und Aufarbeitung der Peptidamidase aus Xanthomonas maltophilia

1.1 Wachstum

In dem partiell optimierten Medium der folgenden Zusammensetzung wurden 40 g Biofeuchtmasse/l und eine Aktivität von 4 U/l erhalten. Die Medienoptimierung erfolgte mit Hilfe des Genetischen Algorithmus. Das Nährmedium wurde autoklaviert, Glucose, N-Ac-DL-Met-$NH_2$, $CaCl_2$*2$H_2O$, $MgSO_4$*7$H_2O$ und die Vitaminlösung wurden steril hinzugefügt.

| | |
|---|---|
| N-Ac-D,L-Met-$NH_2$ | 4.3 g/l |
| Hefeextrakt | 4.5 g/l |
| Pepton aus Casein | 19.7 g/l |
| Glukose | 18.4 g/l |
| $KH_2PO_4$ | 0.5 g/l |
| $K_2HPO_4$ | 2.0 g/l |
| NaCl | 1.0 g/l |
| CaCl2*2 $H_2O$ | 0.05 g/l |
| $MgSO_4$*7 $H_2O$ | 0.1 g/l |
| Vitaminlösung nach Schlegel* | 2.5 ml/l |
| Spurensalzlösung | 0.8 ml/l |
| (Agar bei Festmedien | 18.0 g/l) |

* Schlegel, H. G. (1985): Allgemeine Mikrobiologie, Thieme Verlag, Stuttgart

| | | |
|---|---|---|
| Spurensalzlösung | $H_3BO_3$ | 75.00 mg |
| | $MnCl_2*4H_2O$ | 50.00 mg |
| | $ZnCl_2$ | 187.50 mg |
| | $CuSO_4*5H_2O$ | 50.00 mg |
| | $FeCl_3*6H_2O$ | 625.00 mg |
| | $(NH_4)_6Mo_7O_{24}*4H_2O$ | 25.00 mg |
| | $CoCl_2*6H_2O$ | 37.50 mg |
| | $NiCl_2*6H_2O$ | 50.00 mg |
| | $H_2O$ | ad 0.2 l |

1.2 Zellaufschluß

Der Zellaufschluß erfolgte durch Naßvermahlung einer 20 - 40 %igen Zellsuspension in 50 mM Tris/HCl-Puffer, pH 7.5 mit Glasperlen Ø 0.3 mm. Die Glasperlen sowie die Zelltrümmer wurden durch Zentrifugation abgetrennt. Anschließend wurden die Glasperlen in 50 mM Tris/HCl, pH 7.5 resuspendiert und erneut durch Zentrifugation abgetrennt. Die Überstände wurden vereinigt und stellten den Rohextrakt für die im folgenden beschriebene Aufarbeitung dar. Der Zellaufschluß wurde durch Bestimmung der freigesetzten Proteinmenge nach Bradford (Bradford, M. M. (1976), Anal. Biochem., 72, 248 - 254) und mikroskopische Beobachtung beurteilt.

1.3 Reinigung

1.3.1 Ionenaustauschchromatographie

Als erster Schritt der Aufreinigung der Peptidamidäse aus Xanthomonas maltophilia wurde eine Anionenaustauschchromatographie an Q-Sepharose Fast Flow (Pharmacia, Uppsala) durchgeführt. Für den Anionenaustauscher galten folgende Bedingungen:

| | |
|---|---|
| Säule | 10 cm * π * 1.3 * 1.3 * $cm^2$ = 53 ml Q Sepharose FF (Pharmacia, Uppsala) |
| Laufgeschwindigkeit | 10 ml/min |
| Äquilibrierung | 50 mM Tris, 20 mM KCl, pH 8.0 |
| Probeaufgabe | 77.2 ml Rohextrakt à 6.8 mg BSAeq/ml |
| Waschen | 50 mM Tris/HCl, 20 mM KCl, pH 8.0 |
| Elution | Linearer Gradient mit steigendem Salzgehalt 200 ml 50 mM Tris, 20 mM KCl, pH 8.0 200 ml 50 mM Tris, 200 mM KCl, pH 8.0 |

Die Detektion erfolgte bei 280 nm, die Fraktionen wurden zu je 10 ml aufgefangen. Die Peptidamidase konnte bei einem Salzgehalt von 80 - 120 mM KCl von dem Anionenaustauscher eluiert werden. Die aktiven Fraktionen wurden gepoolt und mit Hilfe einer Amicon Ultrafiltrationszelle mit einer YM 10 Membran auf 2 ml für die anschließende Gelfiltration aufkonzentriert.

1.3.2 Gelfiltration

Die aktiven Fraktionen der Anionenaustauschchromatographie wurden wie beschrieben aufkonzentriert und einer Gelfiltration an Superdex G 75 Material (Pharmacia, Uppsala) unterzogen. Für die Gelfiltration galten folgende Bedingungen:

| | |
|---|---|
| Säule | 60 cm * π * 0.8 * 0.8 * $cm^2$ = 120.6 ml Superdex G 75 (Pharmacia, Uppsala) |
| Laufgeschwindigkeit | 1 ml/min |
| Äquilibrierung | 50 mM Tris, 150 mM KCl, pH 7.5 |
| Probe | 2 ml à 4.55 mg BSAeq/ml der aufkonzentrierten aktiven Fraktionen der Anionenaustauschchromatographie |

(fortgesetzt)

| | |
|---|---|
| Elution | 50 mM Tris, 150 mM KCl, pH 7.5 |
| Fraktionsgröße | 1 ml |
| Detektion | 280 nm |

Die aktiven Fraktionen der Gelfiltration wurden gepoolt und über eine YM 10 Membran in einer Amicon Ultrafiltrationszelle auf 1 ml aufkonzentriert (1.47 mg BSAeq/ml) und standen so für die anschließende isoelektrische Fokussierung zur Verfügung.

1.3.3 Isoelektrische Fokussierung

Die aktiven und wie unter 1.3.2 beschrieben auf 1 ml eingeengten Fraktionen der Gelfiltration wurden mit Hilfe der isoelektrischen Fokussierung weiter aufgereinigt. Die Bedingungen hierfür sind im folgenden wiedergegeben.

| | |
|---|---|
| Säule | 5.1 cm * 0.25 * 0.25 * $cm^2$ * $\pi$ = 1 ml Mono P (Pharmacia, Uppsala) |
| Laufgeschwindigkeit | 1 ml/min |
| Äquilibrierung | 25 mM Triethanolamin, pH 8.0 |
| Probe | 1 ml der aufkonzentrierten aktiven Fraktionen der Gelfiltration (umgepuffert mit 25 mM Triethanolamin, pH 8.0, 1.47 mg BSAeq/ml) |
| Waschen | 25 mM Triethanolamin, pH 8.0 |
| Elution | 10 mM linearer pH-Gradient von A nach B<br>Eluent A: 25 mM Triethanolamin, pH 8.0<br>Eluent B: Polypuffer 74 (1:10 verdünnt, Pharmacia), pH 5.0 |
| Fraktionen | 0.5 ml |
| Detektion | 280 nm |

In den ersten beiden Schritten der Aufreinigung (Anionenaustauschchromatographie und Gelfiltration) wurden störende Protease/Peptidase-Aktivitäten vollständig entfernt. Nach der isoelektrischen Fokussierung wurde ein 533fach angereichertes Präparat erhalten. Im nativen Gel wird eine Hauptbande beobachtet, die enzymatisch aktiv ist. Die N-terminale Sequenz wurde nach Elution der Bande aus dem nativen Gel mittels eines Flüssigphasensequenators (Applied Biosystems 470 mit on-line HPLC-Kopplung) bestimmt.

Beispiel 2

Charakterisierung des Enzyms

pH-Optimum und -Stabilität

In Figur 1 wird die Abhängigkeit der Umsatzrate in % vom pH-Wert dargestellt. In dem pH-Bereich von 3.0 - 7.25 wurde 50 mM Mc-Ilvain-Puffer eingesetzt, zwischen pH 7.0 - 9.0 50 mM Tris/HCl-Puffer und für den basichen pH-Bereich von 9.5 - 10.5 50 mM $Na_2CO_3$-Puffer. Der Testansatz von 1 ml setzte sich wie folgt zusammen:

| | |
|---|---|
| 100 µl | 100 mM Z-Gly-Tyr-$NH_2$ (gelöst in $H_2O$/Dimethylformamid im Verhältnis 1:1) |
| 200 µl | Enzymlösung à 63 µgBSAeq/ml der Reinigungsstufe nach der Gelfiltration |
| 700 µl | Puffer |

Es erfolgte eine Vorinkubation ohne Zugabe von Substrat für 5 min bei 30 °C. Anschließend wurde durch Zugabe von 100 µl Substrat die Reaktion gestartet und 4 Stunden bei 30 °C inkubiert. Zum Abstoppen der Reaktion wurden 100 µl des Reaktionsansatzes entnommen und mit 100 µl Eisessig sowie 1.4 ml HPLC-Laufmittel versetzt (siehe 1.4 Standard Assay der Peptidamidase).

In Fig. 1 sind die Ergebnisse zur Untersuchung des pH-Optimums wiedergegeben. Das pH-Optimum der Peptidamidase aus Xanthomonas maltophilia ergibt zu 6.0 + 0.5.

Für die pH-Stabilität der Peptidamidase aus Xanthomonas maltophilia wurde die relative Aktivität [%] in Abhängigkeit von der Zeit [h] bestimmt. Für den pH-Bereich von 5.0 - 7.0 wurde 50 mM $Kp_i$-Puffer und zwischen 7.0 - 9.0 50 mM Tris/HCl-Puffer eingesetzt. Für die Untersuchungen zur pH-Stabilität wurden 100 µl Enzymlösung à 0.73 mgBSAeq/ml (Reinigungsstufe nach der Gelfiltration) mit 900 µl Puffer unterschiedlichen pH-Wertes bei 30 °C inkubiert. Es wurden zu verschiedenen Zeiten Proben gezogen und die Aktivität mit Z-Gly-Tyr-$NH_2$ als Substrat bestimmt. Die Ergebnisse sind in Fig. 2 dargestellt. Es ergibt sich eine gute Stabilität im Bereich pH 7 - pH 8.

Beispiel 3

Temperaturoptimum und -stabilität

Es wurde das Temperaturoptimum der Peptidamidase aus Xanthomonas maltophilia bestimmt. Dazu wurden 200 µl Enzymlösung à 63 µgBSAeq/ml der Reinigungsstufe nach der Gelfiltration mit 700 µl des auf die jeweilige Temperatur vortemperierten 50 mM Tris/HCl-Puffer, pH 7.5 versetzt und die Reaktion mit 100 µl Z-Gly-Tyr-$NH_2$ (10 mM im Testansatz) gestartet. Aliquots von 100 µl wurden nach 2 h mit 100 µl Eisessig gestoppt und die Aktivität entsprechend 1.4 bestimmt. Das Ergebnis ist in Fig. 3 wiedergegeben. Das Temperaturoptimum lag bei 35 - 40 °C bei einem pH von 7.5.

Für die Temperaturstabilität der Peptidamidase aus Xanthomonas maltophilia wurde die relative Aktivität [%] in Abhängigkeit von der Zeit [h] bestimmt. Dazu wurden 100 µl Enzymlösung à 0.73 mgBSAeq/ml mit 900 µl vortemperierten 50 mM Tris/HCl-Puffer, pH 7.5 versetzt und bei 20, 30, 37 und 56 °C inkubiert. Zu unterschiedlichen Zeiten wurden Proben gezogen und die Aktivität mit Z-Gly-Tyr-$NH_2$ als Substrat bestimmt. Die Ergebnisse sind in Fig. 4 dargestellt. Während das Enzym bei 56 °C bereits nach wenigen Minuten inaktiviert wird, erweist es sich bei 20, 30 und 37 °C als äußerst stabil.

Beispiel 4

Enzymkinetik der Peptidamidase

Die Abhängigkeit der Reaktionsgeschwindigkeit der Peptidamidase aus Xanthomonas maltophilia von der Substratkonzentration (Z-Gly-Tyr-$NH_2$) wurde bestimmt und aus den Daten die kinetischen Parameter nach Marquardt bestimmt. Dazu wurden 50 µl Enzymlösung à 27 µgBSAeq/ml mit 400 µl 50 mM Tris/HCl-Puffer, pH 7.5 versetzt. Die Reaktion wurde durch Zugabe von Z-Gly-Tyr-$NH_2$ als Substrat gestartet. In den Reaktionsansätzen lag das Substrat in einem Konzentrationsbereich von 0.4 bis 20 mM vor. Die Inkubation erfolgte für 2 Stunden bei 30 °C. Anschließend wurde die Enzymaktivität wie unter 1.4 beschrieben bestimmt. Der $K_m$-Wert wurde mit 0.82 mM und $v_{max}$ mit 0.53 U/mg BSAeq bestimmt.

In Figur 5 ist die Reaktionsgeschwindigkeit der Desamidierung von Z-Gly-Tyr-$NH_2$ als Funktion der Substratkonzentration dargestellt.

1.4 Standard Assay der Peptidamidase

Die Reaktionsbedingungen wurden im Hinblick auf die pH-Stabilität der Peptidamidase aus Xanthomonas maltophilia ausgewählt.

Standardassay der Peptidamidase

| | |
|---|---|
| 50 mM Tris/HCl, pH 7.5 | 350 - 700 µl |
| Enzymlösung | 100 - 200 µl |
| 100 mM Z-Gly-Tyr-$NH_2$, gelöst in Puffer/DMF 1:1 (im Test 10 mM) | 50 - 100 µl |
| Testansatz | 500 - 1000 µl |
| Temperatur | 30 °C |
| Inkubationszeit | variabel |

Zum Abstoppen der Reaktion wurden 100 µl der Reaktionslösung entnommen, mit 100 µl Eisessig versetzt und mit 1.4 ml HPLC-Laufmittel aufgefüllt. Ein Aliquot von 20 µl wurde mittels HPLC analysiert. Die Bedingungen für die HPLC-Analytik von Z-Gly-Tyr-$NH_2$ und Z-Gly-Tyr-OH sind nachfolgend aufgeführt.

| | |
|---|---|
| Laufmittel: | 65 % 10 mM TBA (Tetrabutylammoniumsulfat)<br>35 % Acetonitril |

(fortgesetzt)

| | | |
|---|---|---|
| Laufgeschwindigkeit | 1 ml/min | |
| Laufzeit | 10 min | |
| Detektion | 280 nm | |
| Elution | isokratisch | |
| Säule | RP-18, ODS Hypersil (5 (µm) | |
| Retentionszeiten | Z-Gly-Tyr-$NH_2$ | 4.5 min |
| | Z-Gly-Tyr-OH | 5.9 min |

Beispiel 5

Substratspektrum

Für das Substratspektrum der Peptidamidase aus Xanthomonas maltophilia wurden 18 mU der Peptidamidase (Reinigungsstufe nach der Gelfiltration) pro ml Testansatz eingesetzt. Die Konzentration der getesteten Substrate betrug 10 mM im Reaktionsansatz. Soweit nicht anders angegeben, wurden L-Aminosäurederivate eingesetzt. Die Inkubation erfolgte in 50 mM Tris/HCl, pH 7.5 bei 30 °C für 3 Stunden. Die Reaktion wurde durch fünfminütiges Erhitzen bei 95 °C abgestoppt. Die Aktivitätsbestimmung erfolgte mittels enzymatischer Bestimmung des freigesetzten Ammoniaks (Bergmeyer, U. (1985): Methods of enzymatic analysis, S. 459, VCH Verlagsgesellschaft, Weinheim). Tabelle 4a gibt die Umsetzung von Dipeptidamiden wieder, Tabelle 4b die Umsetzung von N-Acetylaminosäureamiden und Tabelle 4c den Einfluß der N-terminalen Schutzgruppen bzw. der zum C-Terminus benachbarten Aminosäure.

Für die Analytik der in Tabelle 4d aufgeführten Substrate und Produkte wurde die folgende dünnschichtchromatographische Methode verwendet. Dazu wurden 1 µl des abgestoppten Reaktionsansatzes aufgetragen.

| | |
|---|---|
| Stationäre Phase | DC-Alufolie Kieselgel 60 $F_{254}$ (20 * 10 $cm^2$) |
| Mobile Phase | Pyridin/Butanol/Eisessig/Wasser (12:15:3:5) |
| Detektion | Ninhydrin (0.3 % in Propan-1-ol) Die Farbentwicklung erfolgte für ca. 3 min bei 100 °C |

Für die Analytik der in Tabelle 4e wiedergegebenen Umsetzung längerkettiger Peptide galten folgende HPLC Trennbedingungen:

| **Substrat** | **Laufmittel*** | **Retentionszeit Amid Säure [min]** | | **Wellenlänge [nm]** |
|---|---|---|---|---|
| Z-Pro-Leu-Gly-$NH_2$ | 60/40 | 6.0 | 7.3 | 256 |
| Z-Gly-Gly-Leu-$NH_2$ | 60/40 | 4.9 | 6.0 | 220 |
| Gly-D-Phe-Tyr-$NH_2$ | 80/20 | 3.8 | 4.4 | 280 |
| Leu-Enkephalinamid | 80/20 | 6.1 | 7.6 | 220 |

* 10 mM Tetrabutylammoniumsulfat/Acetonitril

Zusätzlich wurde mit Hilfe der Dünnschichtchromatographie auf mögliche Protease- bzw. Peptidaseaktivität hin untersucht. Es konnte bei keinem der Peptide eine Freisetzung von Aminosäuren detektiert werden.

Tabelle 4a

| Umsetzung von Dipeptidamiden | |
|---|---|
| **Substrat [10 mM]** | **Relativer Umsatz [%]** |
| Ala-Gly-$NH_2$ | 39 |
| Phe-Gly-$NH_2$ | 51 |
| Tyr-Gly-$NH_2$ | 54 |
| Leu-Gly-$NH_2$ | 54 |

Tabelle 4a (fortgesetzt)

| Umsetzung von Dipeptidamiden | |
|---|---|
| **Substrat [10 mM]** | **Relativer Umsatz [%]** |
| Gly-Gly-$NH_2$ | 8 |
| Val-Gly-$NH_2$ | 22 |
| Pro-Gly-$NH_2$ | 66 |
| | |
| Gly-Tyr-$NH_2$ | 174 |
| | |
| Phe-Ala-$NH_2$ | 203 |
| Ala-Ala-$NH_2$ | 211 |
| Leu-Ala-$NH_2$ | 217 |
| | |
| Gly-Phe-$NH_2$ | 214 |
| Tyr-Phe-$NH_2$ | 190 |
| Ser-Phe-$NH_2$ | 217 |
| Ala-Phe-$NH_2$ | 218 |
| Leu-Phe-$NH_2$ | 203 |
| Val-Phe-$NH_2$ | 140 |
| His-Phe-$NH_2$ | 137 |
| Pro-Phe-$NH_2$ | 158 |
| | |
| Tyr-Pro-$NH_2$ | 0 |
| | |
| Ala-Asn-OH | 0 |
| Gly-Asn-OH | 0 |
| Ala-Gln-OH | 0 |

Tabelle 4b

| Umsetzung von N-Acetylaminosäureamiden | |
|---|---|
| **Substrat [10 mM]** | **Relativer Umsatz [%]** |
| Ac-Ala-$NH_2$ | 182 |
| Ac-Val-$NH_2$ | 1 |
| Ac-Ile-$NH_2$ | 6 |
| Ac-D,L-Neopentylglycinamid | 131 |
| Ac-Pro-$NH_2$ | 0 |
| | |
| Ac-Cys-$NH_2$ | 82 |
| Ac-D,L-Met-$NH_2$ | 102 |
| | |

Tabelle 4b (fortgesetzt)

| Umsetzung von N-Acetylaminosäureamiden | |
|---|---|
| **Substrat [10 mM]** | **Relativer Umsatz [%]** |
| Ac-Ser-$NH_2$ | 58 |
| Ac-Thr-$NH_2$ | 21 |
| | |
| Ac-Phe-$NH_2$ | 134 |
| Ac-Tyr-$NH_2$ | 123 |
| Ac-Phg-$NH_2$ * | 28 |
| Ac-Trp-$NH_2$ | 157 |
| Ac-Nal-$NH_2$* | 84 |
| | |
| Ac-Lys-$NH_2$ | 175 |
| Ac-Arg-$NH_2$ | 110 |
| Ac-His-$NH_2$ | 177 |
| | |
| Ac-Glu-$NH_2$ | 162 |

* Phg = Phenylglycin Nal = Naphthylalanin

Tabelle 4c

| Einfluß der Schutzgruppe bzw. benachbarten Aminosäure auf die Desamidierung des C-Terminus | |
|---|---|
| **Substrat [10 mM]** | **Relativer Umsatz [%]** |
| Ac-Tyr-$NH_2$ | 123 |
| Bz-Tyr-$NH_2$ | 173 |
| Gly-Tyr-$NH_2$ | 174 |
| | |
| Phe-Ala-$NH_2$ | 203 |
| Ala-Ala-$NH_2$ | 211 |
| Leu-Ala-NH2 | 217 |
| Z-Ala-$NH_2$ | 62 |
| Z-D-Ala-$NH_2$ | 0 |
| | |
| N-Methoxy-D,L-Met-$NH_2$ | 109 |
| N-Ethoxy-D,L-Met-$NH_2$ | 124 |
| N-Carbamoyl-D,L-Met-$NH_2$ | 77 |
| N-Acetyl-D,L-Met-$NH_2$ | 102 |
| Arg-Met-$NH_2$ | 125 |

Tabelle 4d

| Enzymatische Umsetzung ungeschützter Aminosäureamide | |
|---|---|
| **Substrat [10 mM]** | **Umsatz** |
| H-Ala-$NH_2$ | 0 |
| H-Arg-$NH_2$ | 0 |
| H-Leu-$NH_2$ | 0 |
| H-Tyr-$NH_2$ | 0 |
| H-Met-$NH_2$ | 0 |
| H-Phe-$NH_2$ | 0 |
| H-Lys-$NH_2$ | 0 |
| H-Val-$NH_2$ | 0 |
| H-Ile-$NH_2$ | 0 |
| H-Thr-$NH_2$ | 0 |
| H-Trp-$NH_2$ | 0 |
| H-Ser-$NH_2$ | 0 |
| | |
| H-D-Ala-$NH_2$ | 0 |
| H-D-Leu-$NH_2$ | 0 |

Tabelle 4e

| Enzymatische Umsetzung längerkettiger Peptide | |
|---|---|
| **Substrat [10 mM]** | **Relativer Umsatz [%]** |
| Z-Pro-Leu-Gly-$NH_2$ | 50 |
| Z-Gly-Gly-Leu-$NH_2$ | 6 |
| Gly-Phe-D-Phe-$NH_2$ | 0 |
| Gly-D-Phe-Tyr-$NH_2$ | 33 |
| Leu-Enkephalinamid | 52 |

Die Ergebnisse zeigen, daß mit Ausnahme von L-Pro alle proteinogenen geschützten Aminosäureamide bzw. Peptidamide desamidiert werden. Auch einige geschützte nichtproteinogene Aminosäureamide werden hydrolysiert (Phenylglycin, Naphthylalanin, Neopentylglycin). D-Aminosäuren in der C-terminalen Position werden nicht umgesetzt. Die Amidfunktion in den Seitenketten von Asparagin und Glutamin werden nicht angegriffen. Die Peptidbindungen längerkettiger Oligopeptide werden nicht gespalten, ungeschützte Aminosäureamide werden nicht umgesetzt.

Beispiel 6

Racematspaltung von N-geschützten Aminosäureamiden

Die Untersuchungen zur Racematspaltung erfolgten in 50 mM Triethylammoniumcarbonat-Puffer, pH 7.5. Die Testansätze à 50 ml mit jeweils 20 U Peptidamidase wurden bei 30 °C inkubiert. Als Substrate standen Z-D,L-Ala-$NH_2$, Ac-D,L-Met-$NH_2$ und Ac-D,L-Neopentylglycinamid (Ac-D,L-Npg-$NH_2$) zur Verfügung. Die Konzentration der Substrate im Reaktionsansatz betrug 10 mM. Es wurden zu unterschiedlichen Zeiten Proben entnommen, die Reaktion durch fünfminütiges Erhitzen bei 95 °C abgestoppt und die Enantiomerenreinheit mittels HPLC untersucht. Die Ergebnisse sind nachfolgend wiedergegeben:

| | |
|---|---|
| Z-D-Ala-OH | 0.4 % |
| Z-L-Ala-OH | 99.6 % |
| Ac-D-Met-OH | 0.2 - 0.3 % |
| Ac-L-Met-OH | 99.7 - 99.8 % |
| Ac-D-Npg-OH | 0.2 % |
| Ac-L-Npg-OH | 99.8 % |

Die Ergebnisse werden auch durch Fig. 6, die sich auf die Racematspaltung von Z-D,L-Ala-$NH_2$ bezieht, und durch die Fig. 7, die die enantioselektive Desamidierung von N-Ac-D,L-Met-$NH_2$ darstellt, erläutert.

Beispiel 7

Einfluß von Enzymeffektoren auf die Peptidamidaseaktivität

Die Untersuchungen erfolgten in 50 mM Tris/HCl, pH 7.5. Nach einer Vorinkubation des Enzyms (38 mU pro Ansatz, Reinigungsstufe nach der Gelfiltration) mit dem Effektor (10 mM im Testansatz) für 1 h bei 30 °C wurde die Reaktion durch Zugabe von Substrat (Z-Gly-Tyr-$NH_2$, 10 mM im Testansatz) gestartet. Die Reaktionsansätze wurden für 2.5 h bei 30 °C inkubiert. Tabelle 5 gibt die Ergebnisse zum Einfluß von Enzymeffektoren auf die Peptidamidaseaktivität wieder. Es zeigt sich, daß die Peptidamidase weder zu den klassischen Serinhydrolasen gehört, die bereits bei einer Konzentration an PMSF von 1 mM bzw. an Pefabloc von 0.2 mM gehemmt werden, noch zu den Metalloenzymen, die von EDTA bzw. 1,10-Phenanthrolin in dem hier angegebenen Konzentrationsbereich gehemmt werden.

Tabelle 5

| Enzymatische Aktivität in Gegenwart potentieller Inhibitoren | |
|---|---|
| **Potentieller Inhibitor [10 mM im Test]** | **Restaktivität [%]** |
| $CuSO_4$*5 $H_2O$ | 103 |
| $CoCl_2$*6 $H_2O$ | 80 |
| $NiCl_2$*6 $H_2O$ | 93 |
| $HgCl_2$*6 $H_2O$ | 67 |
| | |
| EDTA | 96 |
| 1,10-Phenanthrolin | 92 |
| 2,2'-Dipyridyl | 97 |
| | |
| D-Cycloserin | 100 |
| Semicarbazid | 99 |
| | |
| 1,4-Dithio-DL-threitol (DTT) | 96 |
| | |
| Actinonin | 106 |
| Phenylmethansulfonylfluorid (PMSF) | 58 |
| 4-(2-Aminoethyl)-benzylsulfonylfluorid<br>(Pefabloc) 10.0 mM<br>0.3 mM<br>0.1 mM | <br>0<br>62<br>82 |

Beispiel 8

Einfluß von Lösungsmitteln auf die Peptidamidaseaktivität und Stabilität der Peptidamidase bei Inkubation in Lösungsmittel

Für die Untersuchungen zum Einfluß von Lösungsmitteln auf die Peptidamidaseaktivität wurden jeweils 200 µl Enzymlösung (59 µg BSAeq/ml, Reinigungsstufe nach der Gelfiltration) mit 700 µl eines entsprechenden Gemisches von 50 mM Tris/Lösungsmittel, pH 7.5 versetzt. Als Lösungsmittel wurden Dimethylformamid (DMF), Aceton, Ethanol und Propan-1-ol getestet, die im Reaktionsansatz bis zu 70 % Volumenanteil vorlagen. Die Reaktion wurde durch Zugabe von Substrat Z-Gly-Tyr-$NH_2$ gestartet. Die Inkubation erfolgte für 90 Minuten bei 30 °C. Anschließend wurden Aliquots von 100 µl der Reaktionsansätze mit 100 µl Eisessig versehen und die Enzymaktivität wie unter 1.4 beschrieben bestimmt. In Fig. 8 sind die Ergebnisse zum Einfluß von Lösungsmitteln auf die Enzymaktivität wiedergegeben. Es zeigt sich, daß bei 20 % DMF noch 94 % Restaktivität vorhanden ist und selbst bei 30 % noch 59 %. In der Gegenwart anderer Lösungsmittel wie Propan-2-ol büßt das Enzym deutlich Aktivität ein, aber ist trotzdem bei einem Gehalt von 10 % nicht vollständig inaktiviert (Restaktivität 30 %).

Zur Bestimmung der Lösungsmittelstabilität der Peptidamidase aus Xanthomonas maltophilia wurden 600 µl Enzymlösung à 57 µg BSAeq/ml (Reinigungsstufe nach der Gelfiltration) mit 150 µl Lösungsmittel bzw. als Kontrolle 150 µl 50 mM Tris/HCl, pH 7.5 versetzt. Die Inkubation erfolgte bei 30 °C. Zu verschiedenen Zeiten wurden Proben für die Aktivitätsbestimmung gezogen. Als Lösungsmittel wurden Dimethylformamid (DMF), Aceton, Ethanol und Propan-1-ol getestet. In Fig. 9 ist die Lösungsmittelstabilität der Peptidamidase aus Xanthomonas maltophilia dargestellt. Nach 26 Stunden ergibt sich bei einem Lösungsmittelgehalt von 20 % DMF noch eine Restaktivität von 32 %.

Beispiel 9

Bestimmung der Molmasse

Die relative Molmasse der Peptidamidase aus Xanthomonas maltophilia wurde durch Gelfiltration bestimmt (Laufbedingungen siehe 1.3.2). Folgende Eichproteine wurden eingesetzt.

| | Molekulargewicht [Dalton] | $V_e$ [ml] | $K_{AV}$ [-] |
|---|---|---|---|
| Ribonuklease A | 13700 | 85.6 | 0.53 |
| Chymotrypsinogen | 25000 | 77.9 | 0.42 |
| Ovalbumin | 43000 | 65.7 | 0.26 |
| Rinderserumalbumin | 67000 | 58.6 | 0.16 |
| Blue Dextran 2000 | 2000000 | 46.4 | 0.00 |

Die Detektion erfolgte bei 280 nm. Das Elutionsvolumen der Peptidamidase wurde durch Aktivitätsbestimmung ermittelt. Aus dem Elutionsvolumen wurde anhand der Eichkurve die relative Molmasse zu 38000 + 1000 Dalton bestimmt.

Beispiel 10

Induzierbarkeit der Peptidamidase aus Xanthomonas maltophilia

Für die Untersuchungen der Induzierbarkeit der Peptidamidase aus Xanthomonas maltophilia wurden 1 l Erlenmeyerkolben mit 200 ml des im 2. Teil des Screenings benutzten Nährmediums versehen. Dabei wurde der Anteil des Hefeextraktes auf 0.1 % erhöht und jeweils nur ein Amidderivat zu 0.5 % dem Medium zugesetzt. Zusätzlich wurden Medien ohne Zusatz von Amiden bzw. mit 1.5 % Hefeextrakt untersucht. Als Amide wurden Ac-DL-Met-$NH_2$, Leucinamid und Carnitinamid eingesetzt. Die Kolben wurden 0.5 %ig angeimpft und 2 Tage bei 30 °C und 120 Upm geschüttelt. Die Gewinnung der Rohextrakte erfolgte wie bereits oben beschrieben. In Tabelle 6 sind die Ergebnisse wiedergegeben. Aus den Daten geht hervor, daß das Enzym zwar auch ohne Zugabe von Ac-DL-Met-$NH_2$ als Induktor in der Zelle gebildet wird, daß aber die spezifische Aktivität durch Zugabe dieses Induktor verdoppelt bis verdreifacht werden kann. Die Zugabe anderer Amide zeigt hingegen keinen Einfluß.

Tabelle 6

| Induktion der Peptidamidase aus Xanthomonas maltophilia | | | |
|---|---|---|---|
| **Zusätze** | **Aktivität [mU/ml]** | **Proteingehalt [mg BSAeq/ml]** | **Spez. Aktivität [mU/mg BSAeq]** |
| Ohne Zusatz | 29 | 6.62 | 4 |
| 1.5 % Hefeextrakt | 58 | 8.29 | 7 |
| Ac-DL-Met-$NH_2$ | 88 | 6.65 | 13 |
| Leu-$NH_2$ | 15 | 5.22 | 3 |
| Carnitinamid | 30 | 7.13 | 7 |

Beispiel 11

Darstellung racemischer $N_\alpha$-geschützter Aminosäureamide für die enzymatische Trennung mit Peptidamidase

A) $N_\alpha$-Formyl-DL-methioninamid 30 g (0,20 mol) DL-Methioninamid wurden mit 250 ml Ameisensäuremethylester zwei Tage bei Raumtemperatur gerührt, wobei sich feine, leicht gelbliche Kristalle bildeten. Diese wurden abfiltriert und aus einem Gemisch von 150 ml Hexan und 160 ml Ethanol umkristallisiert. Nach Abkühlen, Filtrieren, Waschen und Trocknen wurden 23 g (55 %) $N_\alpha$-Formyl-DL-methioninamid in Form farbloser Kristalle vom Schmelzpunkt 121 - 122 °C erhalten.

B) $N_\alpha$-Methylaminocarbonyl-DL-methioninamid Zu einer Lösung von 30 g (0,2 mol) DL-Methioninamid in 350 ml Wasser wurden bei 5 - 10 °C 18 ml (0,3 mol) Methylisocyanat getropft, wobei sich ein farbloser, kristalliner Niederschlag bildete. Nach 30 min Nachrühren bei 5 - 10 °C und 30 min bei Raumtemperatur wurden die Kristalle abfiltriert, mit Wasser gewaschen und getrocknet, wobei 25 g Produkt erhalten wurden. Aufkonzentrieren der Mutterlauge lieferte weitere 10 g. Durch Umkristallisation aus Methanol/Essigester wurden 29 g (70 %) $N_\alpha$-Methylaminocarbonyl-DL-methioninamid in Form leichter, flockiger Kristalle vom Schmelzpunkt 156 - 157 °C erhalten.

C) $N_\alpha$-Methoxycarbonyl-DL-methioninamid Zu einer Lösung von 150 g (1,02 mol) DL-Methioninamid in 200 ml Wasser wurden bei 5 - 10 °C 85 ml (1,11 mol) Methylchlorformiat getropft, wobei der pH-Wert durch Zugabe von Natronlauge bei 8 - 10 gehalten wurde. Nach 30 min Nachreaktion wurden 60 ml Wasser zugesetzt, worauf sich Kristalle bildeten, die abfiltriert, mit Wasser gewaschen und in 250 ml Wasser gelöst wurden. Diese Lösung wurde zweimal und die Kristallisationsmutterlauge einmal mit je 500 ml Methylenchlorid extrahiert. Nach Einrotieren verblieben 162 g gelblich-kristalliner Rückstand. Dieser wurde in 200 ml heißem Ethanol gelöst. Dann wurden 400 ml und nach Erscheinen der ersten farblosen Kristalle nochmals 200 ml MTBE zugesetzt. Nach Kühlung im Eisbad, Abfiltrieren, Waschen mit 200 ml MTBE und Trocknung wurden 127 g (60 %) $N_\alpha$-Methoxycarbonyl-DL-methioninamid vom Schmelzpunkt 93 - 94 °C erhalten.

D) $N_\alpha$-Ethoxycarbonyl-DL-methioninamid Zu einer Lösung von 150 g (1,02 mol) DL-Methioninamid in 200 ml Wasser wurden bei 5 - 10 °C 106 ml (1,10 mol) Ethylchlorformiat getropft, wobei der pH-Wert durch Zugabe von Natronlauge bei 7 - 9 gehalten wurde. Es bildete sich schnell ein dicker, feinkristalliner Niederschlag. Durch Zusatz von 800 ml Wasser wurde eine gerade noch rührbare Suspension erhalten, die noch 3 h bei Raumtemperatur gerührt wurde. Anschließend wurden die farblosen Kristalle abfiltriert, mit Wasser gewaschen und aus 900 ml Wasser umkristalliert. Nach Kühlung auf 0 - 5 °C, Abfiltrieren, Waschen mit Wasser und Trocknen wurden 129 g (57 %) $N_\alpha$-Ethoxycarbonyl-DL-methioninamid vom Schmelzpunkt 110 - 112 °C erhalten.

E) $N_\alpha$-Benzyloxycarbonyl-DL-methioninamid
Zu einer Lösung von 50 g (0,34 mol) DL-Methioninamid in 200 ml Wasser wurden bei 5 - 20 °C 51 ml Benzyloxycarbonylchlorid getropft, wobei der pH-Wert mit Natronlauge bei 7 - 9 gehalten wurde. Es bildete sich sofort ein schleimiger Niederschlag, der auf Zusatz von 200 ml MTBE feinkristallin wurde. Nach beendeter Zugabe wurde noch 30 min bei Raumtemperatur gerührt. Die ausgefallenen farblosen Kristalle wurden anschließend abfiltriert, mit wenig MTBE gewaschen und aus 700 ml Toluol umkristallisiert. Nach Abfiltrieren, Waschen mit Toluol und Trocknen wurden 67 g (69 %) $N_\alpha$-Benzyloxycarbonyl-DL-methioninamid vom Schmelzpunkt 120 - 122 °C erhalten.

F) $N_\alpha$-Acetyl-DL-neopentylglycinamid

Zu einer Lösung von 17 g (0,12 mol) DL-Neopentylglycin in 200 ml Wasser wurden unter Eiskühlung in ca. 30 min 12 ml (0,13 mol) Acetanhydrid getropft, wobei der pH-Wert mit Natronlauge bei etwa 8 gehalten wurde. Nach 1 h Rühren bei Raumtemperatur wurde dreimal mit 100 ml Methylenchlorid extrahiert und die organische Phase nach Trocknen über Natriumsulfat einrotiert. Es verblieben 20 g (89 %) N-Acetyl-DL-neopentylglycin als farbloser Feststoff.

Zu 19 g (0,1 mol) N-Acetyl-DL-neopentylglycin in 150 ml THF wurden dann bei -10 °C in 15 min 14 ml (0,1 mol) Triethylamin getropft. Nach 10 min wurde eine Lösung von 10 ml (0,1 mol) Ethylchlorformiat in 10 ml THF so zugetropft, daß die Temperatur -5 °C nicht überschritt. Dann wurden 70 ml 25 %ige Ammoniaklösung auf einmal zugegeben, anschließend 50 ml THF, und dann 2 h bei -5 °C und über Nacht bei Raumtemperatur gerührt. Anschließend wurde der Ansatz zur Trockne eingedampft, mit 100 ml Wasser ausgerührt, der Feststoff abfiltriert und aus 70 ml Methanol umkristallisiert. Es wurden insgesamt 15 g (78 %) $N_\alpha$-Acetyl-DL-neopentylglycinamid in Form farbloser Kristalle mit einem Schmelzpunkt >205 °C erhalten.

G) $N_\alpha$-Benzyloxycarbonyl-DL-neopentylglycinamid

Zu einer Lösung von 15 g (0,10 ml) DL-Neopentylglycin in 150 ml Wasser wurden unter Eiskühlung in ca. 30 min 15 ml (0,11 mol) Benzyloxycarbonylchlorid getropft, wobei der pH-Wert mit Natronlauge bei etwa 9 gehalten wurde. Nach 1 h Rühren bei Raumtemperatur wurde dreimal mit 75 ml Methylenchlorid extrahiert und die organische Phase nach Trocknen über Natriumsulfat einrotiert. Es verblieben 25 g (90 %) N-Benzyloxycarbonyl-DL-neopentylglycin als farbloses Öl, das beim Stehen kristallisierte.

Zu 24 g (0,09 mol) N-Benzyloxycarbonyl-DL-neopentylglycin in 150 ml THF wurden dann bei -10 °C in 15 min 12 ml (0,09 mol) Triethylamin getropft. Nach 10 min wurde eine Lösung von 8 ml (0,09 mol) Ethylchlorformiat in 10 ml THF in ca. 30 min so zugetropft, daß die Temperatur -5 °C nicht überschritt. Nach 30 min Rühren bei -5 °C wurden 60 ml 25 %ige Ammoniaklösung auf einmal zugegeben und dann 2 h bei -5 °C und über Nacht bei Raumtemperatur gerührt. Anschließend wurde der Ansatz zur Trockne eingedampft, mit 50 ml Wasser aufgenommen und mit 150 ml Methylenchlorid extrahiert. Nach Trocknen der organischen Phase über Natriumsulfat und Einrotieren verblieben 26 g Rückstand, die in 50 ml Methanol gelöst und dann innerhalb von ca. 2 h unter Eiskühlung mit 70 ml Wasser versetzt wurden. Nach Filtration, Waschen mit Methanol/Wasser und Trocknen wurden 13 g (54 %) $N_\alpha$-Benzyloxycarbonyl-DL-neopentylglycinamid als farbloser Feststoff mit einem Schmelzbereich von 132 - 137 °C erhalten.

Beispiel 12

Darstellung von D-Neopentylglycin aus N-Acetyl-D,L-Neopentylglycinamid

10 mM N-Acetyl-D,L-Neopentylglycinamid wurden in einem 50 ml Erlenmeyerkolben in 50 mM Triethylammoniumcarbonat-Puffer bei pH 7.5 und 30 °C mit 20 U Peptidamidase inkubiert. Per HPLC wurde der Umsatz kontrolliert. Nach 24 Stunden betrug der Umsatz 50 % und änderte sich über die Zeit nicht mehr. Das Enzym wurde nun durch fünfminütiges Erhitzen bei 95 °C desaktiviert und die Enantiomerenreinheit untersucht. Die Reinheit von N-Acetyl-L-Neopentylglycin (bestimmt durch Ligandenaustauschchromatographie) betrug 99.8 %, die Reinheit des N-Acetyl-D-Neopentylglycinamids (bestimmt durch Inklusionschromatographie) betrug 99.7 %. Durch Anionenaustauschchromatographie an Amberlite M 500 wurden die beiden Produkte getrennt und das N-Acetyl-D-Neopentylglycinamid durch mehrstündige Hydrolyse in 6-normaler siedender Salzsäure in die freie D-Aminosäure überführt, wobei der Nachweis des vollständigen Umsatzes per HPLC oder DC erfolgt. Der Drehwert des D-Neopentylglycins [$\alpha_D^{25}$] (c = 0.5, 6N HCl) betrug -16.2°.

Verwendete Methoden zur Enantiomerentrennung:

Die Enantiomeren des verbleibenden N-Acyl-Aminosäureamids wurden durch Inklusionschromatographie voneinander getrennt, die beiden Enantiomeren des N-Acyl-Aminosäureprodukts durch Ligandenaustauschchromatographie.

| Aktenzeichen des Anmelders oder Anwalts 940041 AM | Internationales Aktenzeichen |
|---|---|

ANGABEN ZU EINEM HINTERLEGTEN MIKROORGANISMUS

(Regel 13bis PCT)

A. Die nachstehenden Angaben betreffen den Mikroorganismus, der in der Beschreibung genannt ist auf Seite 8, Zeile 3 (Tabelle).

**B. KENNZEICHNUNG DER HINTERLEGUNG** — Weitere Hinterlegungen sind auf einem zusätzlichen Blatt gekennzeichnet [X]

Name der Hinterlegungsstelle
DSM - Deutsche Sammlung von Mikroorganismen und Zellkulturen GmbH

Anschrift der Hinterlegungsstelle *(einschließlich Postleitzahl und Land)*
Mascheroder Weg 1b, D-38124 Braunschweig

| Datum der Hinterlegung | Eingangsnummer |
|---|---|
| 02. Mai 1994 (02.05.1994) | DSM 9181 |

**C. WEITERE ANGABEN** *(falls nicht zutreffend, bitte frei lassen)* — Die Angaben werden auf einem gesonderten Blatt fortgesetzt [X]

**D. BESTIMMUNGSSTAATEN, FUR DIE ANGABEN GEMACHT WERDEN** *(falls die Angaben nicht für alle Bestimmungsstaaten gelten)*

**E. NACHREICHUNG VON ANGABEN** *(falls nicht zutreffend, bitte frei lassen)*

Die nachstehenden Angaben werden spater beim Internationalen Büro eingereicht *(bitte Art der Angaben nennen, z. B. "Eingangsnummer der Hinterlegung")*

| Nur zur Verwendung im Anmeldeamt | Nur zur Verwendung im Internationalen Büro |
|---|---|
| [X] Dieses Blatt ist eingegangen mit der internationalen Anmeldung | [ ] Dieses Blatt ist beim Internationalen Büro eingegangen am: |
| Bevollmächtigter Bediensteter<br>Marinus v.d. Nouweland | Bevollmachtigter Bediensteter |

Formblatt PCT/RO/134 (Juli 1992)

**BUDAPESTER VERTRAG ÜBER DIE INTERNATIONALE ANERKENNUNG DER HINTERLEGUNG VON MIKROORGANISMEN FÜR DIE ZWECKE VON PATENTVERFAHREN**

**INTERNATIONALES FORMBLATT**

Degussa AG
GB Industrie- und Feinchemikalien
Forschung und Entwicklung
Postfach 1345
63403 Hanau

**EMPFANGSBESTÄTIGUNG BEI ERSTHINTERLEGUNG,**
**ausgestellt gemäß Regel 7.1 von der unten angegebenen**
**INTERNATIONALEN HINTERLEGUNGSSTELLE**

**I. KENNZEICHNUNG DES MIKROORGANISMUS**

| Vom HINTERLEGER zugeteiltes Bezugszeichen: | Von der INTERNATIONALEN HINTERLEGUNGSSTELLE zugeteilte EINGANGSNUMMER: |
|---|---|
| 11 | DSM 9181 |

**II. WISSENSCHAFTLICHE BESCHREIBUNG UND/ODER VORGESCHLAGENE TAXONOMISCHE BEZEICHNUNG**

Mit dem unter I. bezeichneten Mikroorganismus wurde

( ) eine wissenschaftliche Beschreibung
(X) eine vorgeschlagene taxonomische Bezeichnung

eingereicht.
(Zutreffendes ankreuzen).

**III. EINGANG UND ANNAHME**

Diese internationale Hinterlegungsstelle nimmt den unter I bezeichneten Mikroorganismus an, der bei ihr am 1994-05-02 (Datum der Ersthinterlegung)[1] eingegangen ist.

**IV. EINGANG DES ANTRAGS AUF UMWANDLUNG**

Der unter I bezeichnete Mikroorganismus ist bei dieser Internationalen Hinterlegungsstelle am eingegangen (Datum der Ersthinterlegung) und ein Antrag auf Umwandlung dieser Ersthinterlegung in eine Hinterlegung gemäß Budapester Vertrag ist am eingegangen (Datum des Eingangs des Antrags auf Umwandlung).

**V. INTERNATIONALE HINTERLEGUNGSSTELLE**

| | |
|---|---|
| Name: DSM-DEUTSCHE SAMMLUNG VON MIKROORGANISMEN UND ZELLKULTUREN GmbH<br>Anschrift: Mascheroder Weg 1b<br>D-38124 Braunschweig | Unterschrift(en) der zur Vertretung der internationalen Hinterlegungsstelle befugten Person(en) oder des (der) von ihr ermächtigten Bediensteten:<br>U. Weihs<br>Datum: 1994-05-06 |

[1] Falls Regel 6.4 Buchstabe d zutrifft, ist dies der Zeitpunkt, zu dem der Status einer internationalen Hinterlegungsstelle erworben worden ist.
Formblatt DSM-BP/4 (einzige Seite) 12/93

BUDAPESTER VERTRAG ÜBER DIE INTERNATIONALE ANERKENNUNG DER HINTERLEGUNG VON MIKROORGANISMEN FÜR DIE ZWECKE VON PATENTVERFAHREN

INTERNATIONALES FORMBLATT

Degussa AG
GB Industrie- und Feinchemikalien
Forschung und Entwicklung
Postfach 1345
63403 Hanau

**LEBENSFÄHIGKEITSBESCHEINIGUNG**
ausgestellt gemäß Regel 10.2 von der unten angegebenen
**INTERNATIONALEN HINTERLEGUNGSSTELLE**

| I. HINTERLEGER | II. KENNZEICHNUNG DES MIKROORGANISMUS |
|---|---|
| Name: Degussa AG<br>GB Industrie- und Feinchemikalien<br>Anschrift: Forschung und Entwicklung<br>Postfach 1345<br>63403 Hanau | Von der INTERNATIONALEN HINTERLEGUNGSSTELLE zugeteilte EINGANGSNUMMER:<br>DSM 9181<br><br>Datum der Hinterlegung oder Weiterleitung[1]:<br>1994-05-02 |

**III. LEBENSFÄHIGKEITSBESCHEINIGUNG**

Die Lebensfähigkeit des unter II genannten Mikroorganismus ist am 1994-05-02 [2] geprüft worden.
Zu diesem Zeitpunkt war der Mikroorganismus

(X)[3] lebensfähig
( )[3] nicht mehr lebensfähig

**IV. BEDINGUNGEN, UNTER DENEN DIE LEBENSFÄHIGKEITSPRÜFUNG DURCHGEFÜHRT WORDEN IST[4]**

**V. INTERNATIONALE HINTERLEGUNGSSTELLE**

| | |
|---|---|
| Name: DSM-DEUTSCHE SAMMLUNG VON MIKROORGANISMEN UND ZELLKULTUREN GmbH<br><br>Anschrift: Mascheroder Weg 1b<br>D-38124 Braunschweig | Unterschrift(en) der zur Vertretung der internationalen Hinterlegungsstelle befugten Person(en) oder des (der) von ihr ermächtigten Bediensteten:<br>U. Weiss<br><br>Datum: 1994-05-06 |

[1] Angabe des Datums der Ersthinterlegung. Wenn eine erneute Hinterlegung oder eine Weiterleitung vorgenommen worden ist, Angabe des Datums der jeweils letzten erneuten Hinterlegung oder Weiterleitung.
[2] In den in Regel 10.2 Buchstabe a Ziffer ii und iii vorgesehenen Fällen Angabe der letzten Lebensfähigkeitsprüfung.
[3] Zutreffendes ankreuzen.
[4] Ausfüllen, wenn die Angaben beantragt worden sind und wenn die Ergebnisse der Prüfung negativ waren.

Formblatt DSM-BP/9 (einzige Seite) 12/93

| Aktenzeichen des Anmelders oder Anwalts 94004 I AM | Internationales Aktenzeichen |
|---|---|

ANGABEN ZU EINEM HINTERLEGTEN MIKROORGANISMUS

(Regel 13bis PCT)

A. Die nachstehenden Angaben betreffen den Mikroorganismus, der in der Beschreibung genannt ist auf Seite 8, Zeile 3 (Tabelle).

| | |
|---|---|
| **B. KENNZEICHNUNG DER HINTERLEGUNG** | Weitere Hinterlegungen sind auf einem zusätzlichen Blatt gekennzeichnet [X] |
| Name der Hinterlegungsstelle<br>DSM - Deutsche Sammlung von Mikroorganismen und Zellkulturen GmbH | |
| Anschrift der Hinterlegungsstelle *(einschließlich Postleitzahl und Land)*<br>Mascheroder Weg 1b, D-38124 Braunschweig | |
| Datum der Hinterlegung<br>02. Mai 1994 (02.05.1994) | Eingangsnummer<br>DSM 9182 |

| | |
|---|---|
| **C. WEITERE ANGABEN** *(falls nicht zutreffend, bitte frei lassen)* | Die Angaben werden auf einem gesonderten Blatt fortgesetzt [X] |

**D. BESTIMMUNGSSTAATEN, FÜR DIE ANGABEN GEMACHT WERDEN** *(falls die Angaben nicht für alle Bestimmungsstaaten gelten)*

**E. NACHREICHUNG VON ANGABEN** *(falls nicht zutreffend, bitte frei lassen)*

Die nachstehenden Angaben werden später beim Internationalen Büro eingereicht *(bitte Art der Angaben nennen, z. B. "Eingangsnummer der Hinterlegung")*

| Nur zur Verwendung im Anmeldeamt | Nur zur Verwendung im Internationalen Büro |
|---|---|
| [X] Dieses Blatt ist eingegangen mit der internationalen Anmeldung | [ ] Dieses Blatt ist beim Internationalen Büro eingegangen am: |
| Bevollmächtigter Bediensteter<br>Y. Marinus-v.d. Nouweland | Bevollmächtigter Bediensteter |

Formblatt PCT/RO/134 (Juli 1992)

**BUDAPESTER VERTRAG ÜBER DIE INTERNATIONALE ANERKENNUNG DER HINTERLEGUNG VON MIKROORGANISMEN FÜR DIE ZWECKE VON PATENTVERFAHREN**

**INTERNATIONALES FORMBLATT**

Degussa AG
GB Industrie- und Feinchemikalien
Forschung und Entwicklung
Postfach 1345
63403 Hanau

**EMPFANGSBESTÄTIGUNG BEI ERSTHINTERLEGUNG,**
**ausgestellt gemäß Regel 7.1 von der unten angegebenen**
**INTERNATIONALEN HINTERLEGUNGSSTELLE**

**I. KENNZEICHNUNG DES MIKROORGANISMUS**

| Vom HINTERLEGER zugeteiltes Bezugszeichen: | Von der INTERNATIONALEN HINTERLEGUNGSSTELLE zugeteilte EINGANGSNUMMER: |
|---|---|
| 4 | DSM 9182 |

**II. WISSENSCHAFTLICHE BESCHREIBUNG UND/ODER VORGESCHLAGENE TAXONOMISCHE BEZEICHNUNG**

Mit dem unter I. bezeichneten Mikroorganismus wurde

( ) eine wissenschaftliche Beschreibung
( ) eine vorgeschlagene taxonomische Bezeichnung

eingereicht.
(Zutreffendes ankreuzen).

**III. EINGANG UND ANNAHME**

Diese internationale Hinterlegungsstelle nimmt den unter I bezeichneten Mikroorganismus an, der bei ihr am 1994-05-02 (Datum der Ersthinterlegung)[1] eingegangen ist.

**IV. EINGANG DES ANTRAGS AUF UMWANDLUNG**

Der unter I bezeichnete Mikroorganismus ist bei dieser Internationalen Hinterlegungsstelle am eingegangen (Datum der Ersthinterlegung) und ein Antrag auf Umwandlung dieser Ersthinterlegung in eine Hinterlegung gemäß Budapester Vertrag ist am eingegangen (Datum des Eingangs des Antrags auf Umwandlung).

**V. INTERNATIONALE HINTERLEGUNGSSTELLE**

| | |
|---|---|
| Name: DSM-DEUTSCHE SAMMLUNG VON MIKROORGANISMEN UND ZELLKULTUREN GmbH<br>Anschrift: Mascheroder Weg 1b<br>D-38124 Braunschweig | Unterschrift(en) der zur Vertretung der internationalen Hinterlegungsstelle befugten Person(en) oder des (der) von ihr ermächtigten Bediensteten:<br>Datum: 1994-05-06 |

[1] Falls Regel 6.4 Buchstabe d zutrifft, ist dies der Zeitpunkt, zu dem der Status einer internationalen Hinterlegungsstelle erworben worden ist.

**BUDAPESTER VERTRAG ÜBER DIE INTERNATIONALE ANERKENNUNG DER HINTERLEGUNG VON MIKROORGANISMEN FÜR DIE ZWECKE VON PATENTVERFAHREN**

**INTERNATIONALES FORMBLATT**

Degussa AG
GB Industrie- und Feinchemikalien
Forschung und Entwicklung
Postfach 1345
63403 Hanau

**LEBENSFÄHIGKEITSBESCHEINIGUNG**
ausgestellt gemäß Regel 10.2 von der unten angegebenen
**INTERNATIONALEN HINTERLEGUNGSSTELLE**

| I. HINTERLEGER | II. KENNZEICHNUNG DES MIKROORGANISMUS |
|---|---|
| Name: Degussa AG<br>GB Industrie- und Feinchemikalien<br>Anschrift: Forschung und Entwicklung<br>Postfach 1345<br>63403 Hanau | Von der INTERNATIONALEN HINTERLEGUNGSSTELLE zugeteilte EINGANGSNUMMER:<br>DSM 9182<br><br>Datum der Hinterlegung oder Weiterleitung[1]:<br>1994-05-02 |

**III. LEBENSFÄHIGKEITSBESCHEINIGUNG**

Die Lebensfähigkeit des unter II genannten Mikroorganismus ist am 1994-05-02 [2] geprüft worden.
Zu diesem Zeitpunkt war der Mikroorganismus

(X)[3] lebensfähig
( )[3] nicht mehr lebensfähig

**IV. BEDINGUNGEN, UNTER DENEN DIE LEBENSFÄHIGKEITSPRÜFUNG DURCHGEFÜHRT WORDEN IST[4]**

**V. INTERNATIONALE HINTERLEGUNGSSTELLE**

| | |
|---|---|
| Name: DSM-DEUTSCHE SAMMLUNG VON MIKROORGANISMEN UND ZELLKULTUREN GmbH<br><br>Anschrift: Mascheroder Weg 1b<br>D-38124 Braunschweig | Unterschrift(en) der zur Vertretung der internationalen Hinterlegungsstelle befugten Person(en) oder des (der) von ihr ermächtigten Bediensteten:<br><br>Datum: 1994-05-06 |

[1] Angabe des Datums der Ersthinterlegung. Wenn eine erneute Hinterlegung oder eine Weiterleitung vorgenommen worden ist, Angabe des Datums der jeweils letzten erneuten Hinterlegung oder Weiterleitung.
[2] In den in Regel 10.2 Buchstabe a Ziffer ii und iii vorgesehenen Fällen Angabe der letzten Lebensfähigkeitsprüfung.
[3] Zutreffendes ankreuzen.
[4] Ausfüllen, wenn die Angaben beantragt worden sind und wenn die Ergebnisse der Prüfung negativ waren.

Formblatt DSM-BP/9 (einzige Seite) 12/93

| Aktenzeichen des Anmelders oder Anwalts 94004 I AM | Internationales Aktenzeichen |
|---|---|

## ANGABEN ZU EINEM HINTERLEGTEN MIKROORGANISMUS

(Regel 13bis PCT)

**A.** Die nachstehenden Angaben betreffen den Mikroorganismus, der in der Beschreibung genannt ist auf Seite 8, Zeile 3 (Tabelle).

**B. KENNZEICHNUNG DER HINTERLEGUNG** — Weitere Hinterlegungen sind auf einem zusätzlichen Blatt gekennzeichnet [X]

Name der Hinterlegungsstelle
DSM - Deutsche Sammlung von Mikroorganismen und Zellkulturen GmbH

Anschrift der Hinterlegungsstelle *(einschließlich Postleitzahl und Land)*
Mascheroder Weg Ib, D-38124 Braunschweig

| Datum der Hinterlegung | Eingangsnummer |
|---|---|
| 02. Mai 1994 (02.05.1994) | DSM 9183 |

**C. WEITERE ANGABEN** *(falls nicht zutreffend, bitte frei lassen)* — Die Angaben werden auf einem gesonderten Blatt fortgesetzt [X]

**D. BESTIMMUNGSSTAATEN, FÜR DIE ANGABEN GEMACHT WERDEN** *(falls die Angaben nicht für alle Bestimmungsstaaten gelten)*

**E. NACHREICHUNG VON ANGABEN** *(falls nicht zutreffend, bitte frei lassen)*

Die nachstehenden Angaben werden später beim Internationalen Büro eingereicht *(bitte Art der Angaben nennen, z. B. "Eingangsnummer der Hinterlegung")*

| Nur zur Verwendung im Anmeldeamt | Nur zur Verwendung im Internationalen Büro |
|---|---|
| [X] Dieses Blatt ist eingegangen mit der internationalen Anmeldung | [ ] Dieses Blatt ist beim Internationalen Büro eingegangen am: |
| Bevollmächtigter Bediensteter: Y. Marinus-v.d. Nouweland | Bevollmächtigter Bediensteter |

Formblatt PCT/RO/134 (Juli 1992)

**BUDAPESTER VERTRAG ÜBER DIE INTERNATIONALE ANERKENNUNG DER HINTERLEGUNG VON MIKROORGANISMEN FÜR DIE ZWECKE VON PATENTVERFAHREN**

**INTERNATIONALES FORMBLATT**

Degussa AG
GB Industrie- und Feinchemikalien
Forschung und Entwicklung
Postfach 1345
63403 Hanau

**EMPFANGSBESTÄTIGUNG BEI ERSTHINTERLEGUNG,**
ausgestellt gemäß Regel 7.1 von der unten angegebenen
**INTERNATIONALEN HINTERLEGUNGSSTELLE**

**I. KENNZEICHNUNG DES MIKROORGANISMUS**

| Vom HINTERLEGER zugeteiltes Bezugszeichen: | Von der INTERNATIONALEN HINTERLEGUNGSSTELLE zugeteilte EINGANGSNUMMER: |
|---|---|
| 18 | DSM 9183 |

**II. WISSENSCHAFTLICHE BESCHREIBUNG UND/ODER VORGESCHLAGENE TAXONOMISCHE BEZEICHNUNG**

Mit dem unter I. bezeichneten Mikroorganismus wurde

( ) eine wissenschaftliche Beschreibung
( ) eine vorgeschlagene taxonomische Bezeichnung

eingereicht.
(Zutreffendes ankreuzen).

**III. EINGANG UND ANNAHME**

Diese internationale Hinterlegungsstelle nimmt den unter I bezeichneten Mikroorganismus an, der bei ihr am 1994-05-02 (Datum der Ersthinterlegung)[1] eingegangen ist.

**IV. EINGANG DES ANTRAGS AUF UMWANDLUNG**

Der unter I bezeichnete Mikroorganismus ist bei dieser Internationalen Hinterlegungsstelle am eingegangen (Datum der Ersthinterlegung) und ein Antrag auf Umwandlung dieser Ersthinterlegung in eine Hinterlegung gemäß Budapester Vertrag ist am eingegangen (Datum des Eingangs des Antrags auf Umwandlung).

**V. INTERNATIONALE HINTERLEGUNGSSTELLE**

| | |
|---|---|
| Name: DSM-DEUTSCHE SAMMLUNG VON MIKROORGANISMEN UND ZELLKULTUREN GmbH<br>Anschrift: Mascheroder Weg 1b<br>D-38124 Braunschweig | Unterschrift(en) der zur Vertretung der internationalen Hinterlegungsstelle befugten Person(en) oder des (der) von ihr ermächtigten Bediensteten:<br>Datum: 1994-05-06 |

[1] Falls Regel 6.4 Buchstabe d zutrifft, ist dies der Zeitpunkt, zu dem der Status einer internationalen Hinterlegungsstelle erworben worden ist.

Formblatt DSM-BP/4 (einzige Seite) 12/93

BUDAPESTER VERTRAG ÜBER DIE INTERNATIONALE ANERKENNUNG DER HINTERLEGUNG VON MIKROORGANISMEN FÜR DIE ZWECKE VON PATENTVERFAHREN

INTERNATIONALES FORMBLATT

Degussa AG
GB Industrie- und Feinchemikalien
Forschung und Entwicklung
Postfach 1345
63403 Hanau

LEBENSFÄHIGKEITSBESCHEINIGUNG
ausgestellt gemäß Regel 10.2 von der unten angegebenen
INTERNATIONALEN HINTERLEGUNGSSTELLE

| I. HINTERLEGER | II. KENNZEICHNUNG DES MIKROORGANISMUS |
|---|---|
| Name: Degussa AG<br>GB Industrie- und Feinchemikalien<br>Anschrift: Forschung und Entwicklung<br>Postfach 1345<br>63403 Hanau | Von der INTERNATIONALEN HINTERLEGUNGSSTELLE zugeteilte EINGANGSNUMMER:<br>DSM 9183<br><br>Datum der Hinterlegung oder Weiterleitung[1]:<br>1994-05-02 |

**III. LEBENSFÄHIGKEITSBESCHEINIGUNG**

Die Lebensfähigkeit des unter II genannten Mikroorganismus ist am 1994-05-02 [2] geprüft worden.
Zu diesem Zeitpunkt war der Mikroorganismus

(X)[3] lebensfähig
( )[3] nicht mehr lebensfähig

**IV. BEDINGUNGEN, UNTER DENEN DIE LEBENSFÄHIGKEITSPRÜFUNG DURCHGEFÜHRT WORDEN IST[4]**

**V. INTERNATIONALE HINTERLEGUNGSSTELLE**

| | |
|---|---|
| Name: DSM-DEUTSCHE SAMMLUNG VON MIKROORGANISMEN UND ZELLKULTUREN GmbH<br><br>Anschrift: Mascheroder Weg 1b<br>D-38124 Braunschweig | Unterschrift(en) der zur Vertretung der internationalen Hinterlegungsstelle befugten Person(en) oder des (der) von ihr ermächtigten Bediensteten:<br>U. Weihs<br>Datum: 1994-05-06 |

1 Angabe des Datums der Ersthinterlegung. Wenn eine erneute Hinterlegung oder eine Weiterleitung vorgenommen worden ist, Angabe des Datums der jeweils letzten erneuten Hinterlegung oder Weiterleitung.
2 In den in Regel 10.2 Buchstabe a Ziffer ii und iii vorgesehenen Fällen Angabe der letzten Lebensfähigkeitsprüfung.
3 Zutreffendes ankreuzen.
4 Ausfüllen, wenn die Angaben beantragt worden sind und wenn die Ergebnisse der Prüfung negativ waren.

Formblatt DSM-BP/9 (einzige Seite) 12/93

| Aktenzeichen des Anmelders oder Anwalts 94004 I AM | Internationales Aktenzeichen |
|---|---|

ANGABEN ZU EINEM HINTERLEGTEN MIKROORGANISMUS

(Regel 13^bis PCT)

A. Die nachstehenden Angaben betreffen den Mikroorganismus, der in der Beschreibung genannt ist auf Seite 8, Zeile 3 (Tabelle).

B. **KENNZEICHNUNG DER HINTERLEGUNG** — Weitere Hinterlegungen sind auf einem zusätzlichen Blatt gekennzeichnet [X]

Name der Hinterlegungsstelle
DSM - Deutsche Sammlung von Mikroorganismen und Zellkulturen GmbH

Anschrift der Hinterlegungsstelle *(einschließlich Postleitzahl und Land)*
Mascheroder Weg 1b, D-38124 Braunschweig

| Datum der Hinterlegung | Eingangsnummer |
|---|---|
| 02. Mai 1994 (02.05.1994) | DSM 9184 |

C. **WEITERE ANGABEN** *(falls nicht zutreffend, bitte frei lassen)* — Die Angaben werden auf einem gesonderten Blatt fortgesetzt [X]

D. **BESTIMMUNGSSTAATEN, FÜR DIE ANGABEN GEMACHT WERDEN** *(falls die Angaben nicht für alle Bestimmungsstaaten gelten)*

E. **NACHREICHUNG VON ANGABEN** *(falls nicht zutreffend, bitte frei lassen)*

Die nachstehenden Angaben werden später beim Internationalen Büro eingereicht *(bitte Art der Angaben nennen, z. B. "Eingangsnummer der Hinterlegung")*

| Nur zur Verwendung im Anmeldeamt | Nur zur Verwendung im Internationalen Büro |
|---|---|
| [X] Dieses Blatt ist eingegangen mit der internationalen Anmeldung | [ ] Dieses Blatt ist beim Internationalen Büro eingegangen am: |
| Bevollmächtigter Bediensteter<br>Y. Martinez d. Nouwriend | Bevollmächtigter Bediensteter |

Formblatt PCT/RO/134 (Juli 1992)

**BUDAPESTER VERTRAG ÜBER DIE INTERNATIONALE ANERKENNUNG DER HINTERLEGUNG VON MIKROORGANISMEN FÜR DIE ZWECKE VON PATENTVERFAHREN**

**INTERNATIONALES FORMBLATT**

Degussa AG
GB Industrie- und Feinchemikalien
Forschung und Entwicklung
Postfach 1345
63403 Hanau

**EMPFANGSBESTÄTIGUNG BEI ERSTHINTERLEGUNG,**
ausgestellt gemäß Regel 7.1 von der unten angegebenen
**INTERNATIONALEN HINTERLEGUNGSSTELLE**

**I. KENNZEICHNUNG DES MIKROORGANISMUS**

| | |
|---|---|
| Vom HINTERLEGER zugeteiltes Bezugszeichen:<br>21 | Von der INTERNATIONALEN HINTERLEGUNGSSTELLE zugeteilte EINGANGSNUMMER:<br>DSM 9184 |

**II. WISSENSCHAFTLICHE BESCHREIBUNG UND/ODER VORGESCHLAGENE TAXONOMISCHE BEZEICHNUNG**

Mit dem unter I. bezeichneten Mikroorganismus wurde

( ) eine wissenschaftliche Beschreibung
( ) eine vorgeschlagene taxonomische Bezeichnung

eingereicht.
(Zutreffendes ankreuzen).

**III. EINGANG UND ANNAHME**

Diese internationale Hinterlegungsstelle nimmt den unter I bezeichneten Mikroorganismus an, der bei ihr am 1994-05-02 (Datum der Ersthinterlegung)[1] eingegangen ist.

**IV. EINGANG DES ANTRAGS AUF UMWANDLUNG**

Der unter I bezeichnete Mikroorganismus ist bei dieser Internationalen Hinterlegungsstelle am eingegangen (Datum der Ersthinterlegung) und ein Antrag auf Umwandlung dieser Ersthinterlegung in eine Hinterlegung gemäß Budapester Vertrag ist am eingegangen (Datum des Eingangs des Antrags auf Umwandlung).

**V. INTERNATIONALE HINTERLEGUNGSSTELLE**

| | |
|---|---|
| Name: DSM-DEUTSCHE SAMMLUNG VON MIKROORGANISMEN UND ZELLKULTUREN GmbH<br><br>Anschrift: Mascheroder Weg 1b<br>D-38124 Braunschweig | Unterschrift(en) der zur Vertretung der internationalen Hinterlegungsstelle befugten Person(en) oder des (der) von ihr ermächtigten Bediensteten:<br><br>U. Weiss<br><br>Datum: 1994-05-06 |

[1] Falls Regel 6.4 Buchstabe d zutrifft, ist dies der Zeitpunkt, zu dem der Status einer internationalen Hinterlegungsstelle erworben worden ist.

Formblatt DSM-BP/4 (einzige Seite) 12/93

BUDAPESTER VERTRAG ÜBER DIE INTERNATIONALE ANERKENNUNG DER HINTERLEGUNG VON MIKROORGANISMEN FÜR DIE ZWECKE VON PATENTVERFAHREN

INTERNATIONALES FORMBLATT

Degussa AG
GB Industrie- und Feinchemikalien
Forschung und Entwicklung
Postfach 1345
63403 Hanau

LEBENSFÄHIGKEITSBESCHEINIGUNG
ausgestellt gemäß Regel 10.2 von der unten angegebenen
INTERNATIONALEN HINTERLEGUNGSSTELLE

| I. HINTERLEGER | II. KENNZEICHNUNG DES MIKROORGANISMUS |
|---|---|
| Name: Degussa AG<br>GB Industrie- und Feinchemikalien<br>Anschrift: Forschung und Entwicklung<br>Postfach 1345<br>63403 Hanau | Von der INTERNATIONALEN HINTERLEGUNGSSTELLE zugeteilte EINGANGSNUMMER:<br>DSM 9184<br>Datum der Hinterlegung oder Weiterleitung[1]:<br>1994-05-02 |

**III. LEBENSFÄHIGKEITSBESCHEINIGUNG**

Die Lebensfähigkeit des unter II genannten Mikroorganismus ist am 1994-05-02 [2] geprüft worden.
Zu diesem Zeitpunkt war der Mikroorganismus

(X)[3] lebensfähig
( )[3] nicht mehr lebensfähig

**IV. BEDINGUNGEN, UNTER DENEN DIE LEBENSFÄHIGKEITSPRÜFUNG DURCHGEFÜHRT WORDEN IST[4]**

**V. INTERNATIONALE HINTERLEGUNGSSTELLE**

| | |
|---|---|
| Name: DSM-DEUTSCHE SAMMLUNG VON MIKROORGANISMEN UND ZELLKULTUREN GmbH<br>Anschrift: Mascheroder Weg 1b<br>D-38124 Braunschweig | Unterschrift(en) der zur Vertretung der internationalen Hinterlegungsstelle befugten Person(en) oder des (der) von ihr ermächtigten Bediensteten:<br><br>Datum: 1994-05-06 |

[1] Angabe des Datums der Ersthinterlegung. Wenn eine erneute Hinterlegung oder eine Weiterleitung vorgenommen worden ist, Angabe des Datums der jeweils letzten erneuten Hinterlegung oder Weiterleitung.
[2] In den in Regel 10.2 Buchstabe a Ziffer ii und iii vorgesehenen Fällen Angabe der letzten Lebensfähigkeitsprüfung.
[3] Zutreffendes ankreuzen.
[4] Ausfüllen, wenn die Angaben beantragt worden sind und wenn die Ergebnisse der Prüfung negativ waren.

Formblatt DSM-BP/9 (einzige Seite) 12/93

| Aktenzeichen des Anmelders oder Anwalts 94004 l AM | Internationales Aktenzeichen |
|---|---|

ANGABEN ZU EINEM HINTERLEGTEN MIKROORGANISMUS

(Regel 13bis PCT)

**A.** Die nachstehenden Angaben betreffen den Mikroorganismus, der in der Beschreibung genannt ist auf Seite 8, Zeile 3 (Tabelle).

**B. KENNZEICHNUNG DER HINTERLEGUNG** — Weitere Hinterlegungen sind auf einem zusätzlichen Blatt gekennzeichnet [X]

Name der Hinterlegungsstelle
DSM - Deutsche Sammlung von Mikroorganismen und Zellkulturen GmbH

Anschrift der Hinterlegungsstelle *(einschließlich Postleitzahl und Land)*
Mascheroder Weg lb, D-38124 Braunschweig

| Datum der Hinterlegung | Eingangsnummer |
|---|---|
| 02. Mai 1994 (02.05.1994) | DSM 9185 |

**C. WEITERE ANGABEN** *(falls nicht zutreffend, bitte frei lassen)* — Die Angaben werden auf einem gesonderten Blatt fortgesetzt [X]

**D. BESTIMMUNGSSTAATEN, FÜR DIE ANGABEN GEMACHT WERDEN**
*(falls die Angaben nicht für alle Bestimmungsstaaten gelten)*

**E. NACHREICHUNG VON ANGABEN** *(falls nicht zutreffend, bitte frei lassen)*

Die nachstehenden Angaben werden spater beim Internationalen Büro eingereicht *(bitte Art der Angaben nennen, z. B. "Eingangsnummer der Hinterlegung")*

| Nur zur Verwendung im Anmeldeamt | Nur zur Verwendung im Internationalen Buro |
|---|---|
| [X] Dieses Blatt ist eingegangen mit der internationalen Anmeldung | [ ] Dieses Blatt ist beim Internationalen Büro eingegangen am: |
| Bevollmächtigter Bediensteter | Bevollmächtigter Bediensteter |

Formblatt PCT/RO/134 (Juli 1992)

BUDAPESTER VERTRAG ÜBER DIE INTERNATIONALE ANERKENNUNG DER HINTERLEGUNG VON MIKROORGANISMEN FÜR DIE ZWECKE VON PATENTVERFAHREN

INTERNATIONALES FORMBLATT

Degussa AG
GB Industrie- und Feinchemikalien
Forschung und Entwicklung
Postfach 1345
63403 Hanau

EMPFANGSBESTÄTIGUNG BEI ERSTHINTERLEGUNG, ausgestellt gemäß Regel 7.1 von der unten angegebenen INTERNATIONALEN HINTERLEGUNGSSTELLE

| I. KENNZEICHNUNG DES MIKROORGANISMUS | |
|---|---|
| om HINTERLEGER zugeteiltes Bezugszeichen:<br>22 | Von der INTERNATIONALEN HINTERLEGUNGSSTELLE zugeteilte EINGANGSNUMMER:<br>DSM 9185 |

**II. WISSENSCHAFTLICHE BESCHREIBUNG UND/ODER VORGESCHLAGENE TAXONOMISCHE BEZEICHNUNG**

Mit dem unter I. bezeichneten Mikroorganismus wurde

( ) eine wissenschaftliche Beschreibung
( ) eine vorgeschlagene taxonomische Bezeichnung

eingereicht.
(Zutreffendes ankreuzen).

**III. EINGANG UND ANNAHME**

Diese internationale Hinterlegungsstelle nimmt den unter I bezeichneten Mikroorganismus an, der bei ihr am 1994-05-02 (Datum der Ersthinterlegung)[1] eingegangen ist.

**IV. EINGANG DES ANTRAGS AUF UMWANDLUNG**

Der unter I bezeichnete Mikroorganismus ist bei dieser Internationalen Hinterlegungsstelle am eingegangen (Datum der Ersthinterlegung) und ein Antrag auf Umwandlung dieser Ersthinterlegung in eine Hinterlegung gemäß Budapester Vertrag ist am eingegangen (Datum des Eingangs des Antrags auf Umwandlung).

| V. INTERNATIONALE HINTERLEGUNGSSTELLE | |
|---|---|
| Name: DSM-DEUTSCHE SAMMLUNG VON MIKROORGANISMEN UND ZELLKULTUREN GmbH<br>Anschrift: Mascheroder Weg 1b<br>D-38124 Braunschweig | Unterschrift(en) der zur Vertretung der internationalen Hinterlegungsstelle befugten Person(en) oder des (der) von ihr ermächtigten Bediensteten:<br>V. Weiss<br>Datum: 1994-05-06 |

[1] Falls Regel 6.4 Buchstabe d zutrifft, ist dies der Zeitpunkt, zu dem der Status einer internationalen Hinterlegungsstelle erworben worden ist.

**BUDAPESTER VERTRAG ÜBER DIE INTERNATIONALE ANERKENNUNG DER HINTERLEGUNG VON MIKROORGANISMEN FÜR DIE ZWECKE VON PATENTVERFAHREN**

INTERNATIONALES FORMBLATT

Degussa AG
GB Industrie- und Feinchemikalien
Forschung und Entwicklung
Postfach 1345
63403 Hanau

**LEBENSFÄHIGKEITSBESCHEINIGUNG**
ausgestellt gemäß Regel 10.2 von der unten angegebenen
INTERNATIONALEN HINTERLEGUNGSSTELLE

| I. HINTERLEGER | II. KENNZEICHNUNG DES MIKROORGANISMUS |
|---|---|
| Name: Degussa AG<br>GB Industrie- und Feinchemikalien<br>Anschrift: Forschung und Entwicklung<br>Postfach 1345<br>63403 Hanau | Von der INTERNATIONALEN HINTERLEGUNGSSTELLE zugeteilte EINGANGSNUMMER:<br>DSM 9185<br><br>Datum der Hinterlegung oder Weiterleitung[1]:<br>1994-05-02 |

**III. LEBENSFÄHIGKEITSBESCHEINIGUNG**

Die Lebensfähigkeit des unter II genannten Mikroorganismus ist am 1994-05-02 [2] geprüft worden.
Zu diesem Zeitpunkt war der Mikroorganismus

(X)[3] lebensfähig
( )[3] nicht mehr lebensfähig

**IV. BEDINGUNGEN, UNTER DENEN DIE LEBENSFÄHIGKEITSPRÜFUNG DURCHGEFÜHRT WORDEN IST[4]**

**V. INTERNATIONALE HINTERLEGUNGSSTELLE**

| | |
|---|---|
| Name: DSM-DEUTSCHE SAMMLUNG VON MIKROORGANISMEN UND ZELLKULTUREN GmbH<br><br>Anschrift: Mascheroder Weg 1b<br>D-38124 Braunschweig | Unterschrift(en) der zur Vertretung der internationalen Hinterlegungsstelle befugten Person(en) oder des (der) von ihr ermächtigten Bediensteten:<br>U. Weihs<br><br>Datum: 1994-05-06 |

[1] Angabe des Datums der Ersthinterlegung. Wenn eine erneute Hinterlegung oder eine Weiterleitung vorgenommen worden ist, Angabe des Datums der jeweils letzten erneuten Hinterlegung oder Weiterleitung.
[2] In den in Regel 10.2 Buchstabe a Ziffer ii und iii vorgesehenen Fällen Angabe der letzten Lebensfähigkeitsprüfung.
[3] Zutreffendes ankreuzen.
[4] Ausfüllen, wenn die Angaben beantragt worden sind und wenn die Ergebnisse der Prüfung negativ waren.

Formblatt DSM-BP/9 (einzige Seite) 12/93

| Aktenzeichen des Anmelders oder Anwalts 94004 I AM | Internationales Aktenzeichen |
|---|---|

ANGABEN ZU EINEM HINTERLEGTEN MIKROORGANISMUS

(Regel 13bis PCT)

**A.** Die nachstehenden Angaben betreffen den Mikroorganismus, der in der Beschreibung genannt ist auf Seite 8, Zeile 3 (Tabelle).

**B. KENNZEICHNUNG DER HINTERLEGUNG** — Weitere Hinterlegungen sind auf einem zusätzlichen Blatt gekennzeichnet [X]

Name der Hinterlegungsstelle
DSM - Deutsche Sammlung von Mikroorganismen und Zellkulturen GmbH

Anschrift der Hinterlegungsstelle *(einschließlich Postleitzahl und Land)*
Mascheroder Weg 1b, D-38124 Braunschweig

| Datum der Hinterlegung | Eingangsnummer |
|---|---|
| 02. Mai 1994 (02.05.1994) | DSM 9186 |

**C. WEITERE ANGABEN** *(falls nicht zutreffend, bitte frei lassen)* — Die Angaben werden auf einem gesonderten Blatt fortgesetzt [X]

**D. BESTIMMUNGSSTAATEN, FUR DIE ANGABEN GEMACHT WERDEN** *(falls die Angaben nicht für alle Bestimmungsstaaten gelten)*

**E. NACHREICHUNG VON ANGABEN** *(falls nicht zutreffend, bitte frei lassen)*

Die nachstehenden Angaben werden später beim Internationalen Büro eingereicht *(bitte Art der Angaben nennen, z. B. "Eingangsnummer der Hinterlegung")*

| Nur zur Verwendung im Anmeldeamt | Nur zur Verwendung im Internationalen Büro |
|---|---|
| [X] Dieses Blatt ist eingegangen mit der internationalen Anmeldung | [ ] Dieses Blatt ist beim Internationalen Büro eingegangen am: |
| Bevollmächtigter Bediensteter: Y. Marinus-v.d. Nouweland | Bevollmächtigter Bediensteter |

Formblatt PCT/RO/134 (Juli 1992)

BUDAPESTER VERTRAG ÜBER DIE INTERNATIONALE
ANERKENNUNG DER HINTERLEGUNG VON MIKROORGANISMEN
FÜR DIE ZWECKE VON PATENTVERFAHREN

INTERNATIONALES FORMBLATT

Degussa AG
GB Industrie- und Feinchemikalien
Forschung und Entwicklung
Postfach 1345
63403 Hanau

EMPFANGSBESTÄTIGUNG BEI ERSTHINTERLEGUNG,
ausgestellt gemäß Regel 7.1 von der unten angegebenen
INTERNATIONALEN HINTERLEGUNGSSTELLE

**I. KENNZEICHNUNG DES MIKROORGANISMUS**

| | |
|---|---|
| om HINTERLEGER zugeteiltes Bezugszeichen:<br>42 | Von der INTERNATIONALEN HINTERLEGUNGSSTELLE zugeteilte EINGANGSNUMMER:<br>DSM 9186 |

**II. WISSENSCHAFTLICHE BESCHREIBUNG UND/ODER VORGESCHLAGENE TAXONOMISCHE BEZEICHNUNG**

Mit dem unter I. bezeichneten Mikroorganismus wurde

( ) eine wissenschaftliche Beschreibung
( ) eine vorgeschlagene taxonomische Bezeichnung

eingereicht.
(Zutreffendes ankreuzen).

**III. EINGANG UND ANNAHME**

Diese internationale Hinterlegungsstelle nimmt den unter I bezeichneten Mikroorganismus an, der bei ihr am 1994-05-02 (Datum der Ersthinterlegung)[1] eingegangen ist.

**IV. EINGANG DES ANTRAGS AUF UMWANDLUNG**

Der unter I bezeichnete Mikroorganismus ist bei dieser Internationalen Hinterlegungsstelle am eingegangen (Datum der Ersthinterlegung) und ein Antrag auf Umwandlung dieser Ersthinterlegung in eine Hinterlegung gemäß Budapester Vertrag ist am eingegangen (Datum des Eingangs des Antrags auf Umwandlung).

**V. INTERNATIONALE HINTERLEGUNGSSTELLE**

| | |
|---|---|
| Name: DSM-DEUTSCHE SAMMLUNG VON MIKROORGANISMEN UND ZELLKULTUREN GmbH<br><br>Anschrift: Mascheroder Weg 1b<br>D-38124 Braunschweig | Unterschrift(en) der zur Vertretung der internationalen Hinterlegungsstelle befugten Person(en) oder des (der) von ihr ermächtigten Bediensteten:<br><br>Datum: 1994-05-06 |

[1] Falls Regel 6.4 Buchstabe d zutrifft, ist dies der Zeitpunkt, zu dem der Status einer internationalen Hinterlegungsstelle erworben worden ist.

Formblatt DSM-BP/4 (einzige Seite) 12/93

BUDAPESTER VERTRAG ÜBER DIE INTERNATIONALE ANERKENNUNG DER HINTERLEGUNG VON MIKROORGANISMEN FÜR DIE ZWECKE VON PATENTVERFAHREN

INTERNATIONALES FORMBLATT

Degussa AG
GB Industrie- und Feinchemikalien
Forschung und Entwicklung
Postfach 1345
63403 Hanau

**LEBENSFÄHIGKEITSBESCHEINIGUNG**
ausgestellt gemäß Regel 10.2 von der unten angegebenen
**INTERNATIONALEN HINTERLEGUNGSSTELLE**

| I. HINTERLEGER | II. KENNZEICHNUNG DES MIKROORGANISMUS |
|---|---|
| Name: Degussa AG<br>GB Industrie- und Feinchemikalien<br>Anschrift: Forschung und Entwicklung<br>Postfach 1345<br>63403 Hanau | Von der INTERNATIONALEN HINTERLEGUNGSSTELLE zugeteilte EINGANGSNUMMER:<br>DSM 9186<br>Datum der Hinterlegung oder Weiterleitung[1]:<br>1994-05-02 |

**III. LEBENSFÄHIGKEITSBESCHEINIGUNG**

Die Lebensfähigkeit des unter II genannten Mikroorganismus ist am 1994-05-02 [2] geprüft worden.
Zu diesem Zeitpunkt war der Mikroorganismus

(X)[3] lebensfähig
( )[3] nicht mehr lebensfähig

**IV. BEDINGUNGEN, UNTER DENEN DIE LEBENSFÄHIGKEITSPRÜFUNG DURCHGEFÜHRT WORDEN IST[4]**

**V. INTERNATIONALE HINTERLEGUNGSSTELLE**

| | |
|---|---|
| Name: DSM-DEUTSCHE SAMMLUNG VON MIKROORGANISMEN UND ZELLKULTUREN GmbH<br>Anschrift: Mascheroder Weg 1b<br>D-38124 Braunschweig | Unterschrift(en) der zur Vertretung der internationalen Hinterlegungsstelle befugten Person(en) oder des (der) von ihr ermächtigten Bediensteten:<br>V. Weiss<br>Datum: 1994-05-06 |

1 Angabe des Datums der Ersthinterlegung. Wenn eine erneute Hinterlegung oder eine Weiterleitung vorgenommen worden ist, Angabe des Datums der jeweils letzten erneuten Hinterlegung oder Weiterleitung.
2 In den in Regel 10.2 Buchstabe a Ziffer ii und iii vorgesehenen Fällen Angabe der letzten Lebensfähigkeitsprüfung.
3 Zutreffendes ankreuzen.
4 Ausfüllen, wenn die Angaben beantragt worden sind und wenn die Ergebnisse der Prüfung negativ waren.

Formblatt DSM-BP/9 (einzige Seite) 12/93

## Patentansprüche

1. Verfahren zur Gewinnung von Peptidamidase enthaltenden Mikroorganismen durch Kultivieren von Mikroorganismen haltigen Proben in Nährmedien, die eine Quelle für Kohlenstoff, Stickstoff und Mineralsalze aufweisen, **dadurch gekennzeichnet,** daß man die Proben zuerst in einem Nährmedium, das Amidstickstoff als Stickstoffquelle aufweist, bebrütet und anschließend entstandene Kolonien auf ein Nährmedium überimpft, das N-Acetyl-D,L-Methioninamid enthält, bebrütet und die Mikroorganismen auswählt, die in beiden Nährmedien wachsen und anschließend auf Peptidamidasereaktivität untersucht.

2. Xanthomonas maltophilia, hinterlegt bei der Deutschen Sammlung für Mikroorganismen unter der Nr. DSM 9181.

3. Mikrobielle Peptidamidase befähigt zur selektiven hydrolytischen Abspaltung der freien Aminogruppe am C-terminalen Ende von Peptidamiden, **dadurch gekennzeichnet,** daß sie aus einem gemäß Anspruch 1 gewonnenen Mikroorganismus erhätlich ist.

4. Mikrobielle Peptidamidase befähigt zur selektiven hydrolytischen Abspaltung der freien Aminogruppe am C-terminalen Ende von Peptidamiden, gewonnen aus den Mikroorganismen gemäß Anspruch 2.

5. Mikrobielle Peptidamidase nach Anspruch 3 oder 4, nach Aufreinigung **gekennzeichnet durch** folgende Parameter

   - Abspaltung der C-terminalen Aminogruppe von Peptidamiden und N-terminal geschützten Aminosäureamiden;
   - keine Spaltung von Peptidbindungen;
   - optimaler pH-Wert bei 6,0 ± 0,5;
   - gute Stabilität im pH-Bereich zwischen pH 7 und pH 8;
   - die optimale Temperatur beträgt 35 - 40 °C bei einem pH-Wert von 7,5;
   - Inhibierung von Serinresten durch Hemmstoffe, wie Phenylmethansulfonylfluorid, sowie insbesondere 4-(2-Aminoethylbenzylsulfonylfluorid) (Pefabloc);
   - das Molekulargewicht beträgt etwa 38.000 Dalton (bestimmt durch Gelfiltration);
   - der isoelektrische Punkt liegt bei pH 5,8;
   - eine N-terminale Anfangssequenz:

| | |
|---|---|
| AS 1 | X |
| AS 2 | Arg |
| AS 3 | Asn |
| AS 4 | Val |
| AS 5 | Pro |
| AS 6 | Phe |
| AS 7 | Pro |
| AS 8 | Tyr |
| AS 9 | Ala |
| AS 10 | Glu |
| AS 11 | Thr |
| AS 12 | Asp |
| AS 13 | Val |
| AS 14 | Ala |
| AS 15 | Asp |
| AS 16 | Leu |
| AS 17 | Gln |

   und ihre isozymen Formen.

6. Verwendung der mikrobiellen Peptidamidase nach einem der Ansprüche 3 bis 5 in einem Verfahren zur Herstellung von Peptiden und N-terminal geschützten Aminosäuren der allgemeinen Formel

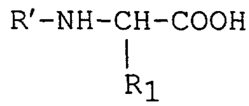

in der R' eine Schutzgruppe oder ein beliebiger peptidisch oder isopeptidisch gebundener Aminosäure- oder Peptidrest ist und $R_1$ Wasserstoff oder eine beliebige Seitenkette bedeutet, zur enzymatischen Abspaltung einer C-terminalen Aminogruppe von einem Peptidamid oder einem N-terminal geschützten Aminosäureamid.

7. Verwendung nach Anspruch 6,
**dadurch gekennzeichnet,**
daß man die enzymatische Reaktion kontinuierlich durchführt.

8. Verwendung nach Anspruch 6 oder 7,
**dadurch gekennzeichnet**,
daß man die Desamidierung als Verfahrensschritt einer gekoppelten Umsetzung mit einem Enzymsystem durchführt, das Proteasen, Peptidasen, Esterasen und/oder Lipasen umfaßt.

9. Verwendung der mikrobiellen Peptidamidase nach einem der Ansprüche 3 bis 5 in einem Verfahren zur Herstellung von Peptiden gemäß Anspruch 6 - 8 durch enzymatische Umsetzung von ggf. N-geschützten Aminosäurealkylestern bzw. ggf. N-geschützten Peptidalkylestern mit Aminosäureamiden in wäßriger Phase oder wäßrig-organischem Milieu, in Gegenwart eines die peptidische Verknüpfung herbeiführenden Enzyms und unter enzymatischer Abspaltung der Amidschutzgruppe, bei welchem Verfahren man die Synthese kontinuierlich ablaufen läßt und das Peptidamid mittels der Peptidamidase enzymatisch zum Peptid hydrolysiert und schließlich das Peptid aufgrund seiner Ladung vom Reaktionsgemisch abtrennt und das Aminosäureamid zurückführt.

10. Verwendung der mikrobiellen Peptidamidase nach einem der Ansprüche 3 bis 5 zur Racematspaltung von N-geschützten Aminosäureamiden, wobei ein racemisches Gemisch von N-geschützten Aminosäureamiden mit der Peptidamidase inkubiert wird und bis zum vollständigen Umsatz des N-geschützten L-Aminosäureamids reagiert wird und nachfolgend die N-geschützte L-Aminosäure vom N-geschützten D-Aminosäureamid aufgrund der Ladungsunterschiede getrennt wird.

11. Verwendung der mikrobiellen Peptidamidase nach einem der Ansprüche 3 bis 5 zur Gewinnung nichtproteinogener D-Aminosäuren, wobei man N-geschützte racemische Aminosäureamide wie N-Acetyl-Neopentylglycinamid, N-Acetyl-Naphthylalaninamid, N-Acetylphenylglycinamid oder ähnliche Derivate einsetzt, die N-Acetyl-L-Aminosäureamide enzymatisch hydrolysiert, die N-Acetyl-D-Aminosäureamide durch Chromatographie aus dem Reaktionsgemisch abtrennt und schließlich mittels Säurehydrolyse in die freien D-Aminosäuren überführt.

**Claims**

1. Process for obtaining microorganisms containing peptide amidase by culturing samples containing microorganisms in nutrient media which comprise a source of carbon, nitrogen and mineral salts,
characterised in that
the samples are first incubated in a nutrient medium which contains amide nitrogen as a source of nitrogen and resultant colonies are then transferred onto a nutrient medium which contains N-acetyl-D,L-methionine amide, incubated and the microorganisms which grow in both nutrient media are selected and then investigated for peptide amidase reactivity.

2. Xanthomonas maltophilia deposited with Deutsche Sammlung für Mikroorganismen under number DSM 9181.

3. Microbial peptide amidase capable of selective hydrolytic elimination of the free amino group on the C-terminal end of peptide amides,
characterised in that

it is obtainable from a microorganism obtained according to claim 1.

**4.** Microbial peptide amidase capable of selective hydrolytic elimination of the free amino group on the C-terminal end of peptide amides obtained from the microorganisms according to claim 2.

**5.** Microbial peptide amidase according to claim 3 or 4 after purification
characterised by
the following parameters

- elimination of the C-terminal amino group from peptide amides and N-terminally protected amino acid amides;
- no cleavage of peptide bonds;
- optimum pH value at 6.0 ± 0.5;
- good stability in the pH range between pH 7 and pH 8;
- optimum temperature is 35-40°C at a pH value of 7.5;
- inhibition of serine residues by inhibitors such as phenylmethanesulfonyl fluoride, as well as in particular 4-(2-aminoethylbenzylsulfonyl fluoride) (Pefabloc);
- molecular weight is approx. 38000 Dalton (determined by gel filtration);
- the isoelectric point is at pH 5.8;
- an N-terminal starting sequence:

| | |
|---|---|
| AA 1 | X |
| AA 2 | Arg |
| AA 3 | Asn |
| AA 4 | Val |
| AA 5 | Pro |
| AA 6 | Phe |
| AA 7 | Pro |
| AA 8 | Tyr |
| AA 9 | Ala |
| AA 10 | Glu |
| AA 11 | Thr |
| AA 12 | Asp |
| AA 13 | Val |
| AA 14 | Ala |
| AA 15 | Asp |
| AA 16 | Leu |
| AA 17 | Gln |

and the isozymic forms thereof.

**6.** Use of the microbial peptide amidase according to one of claims 3 to 5 in a process for the production of peptides and N-terminally protected amino acids of the general formula

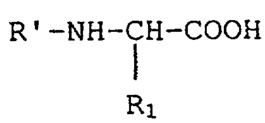

in which R' is a protective group or any desired amino acid or peptide residue attached by a peptide or isopeptide bond and $R_1$ means hydrogen or any desired side chain, for the enzymatic elimination of a C-terminal amino group from a peptide amide or an N-terminally protected amino acid amide.

**7.** Use according to claim 6,

characterised in that
the enzymatic reaction is performed continuously.

**8.** Use according to claim 6 or 7,
characterised in that
the deamidation is performed as a process step of a coupled reaction with an enzyme system which comprises proteases, peptidases, esterases and/or lipases.

**9.** Use of the microbial peptide amidase according to one of claims 3 to 5 in a process for the production of peptides according to claims 6-8 by enzymatic conversion of optionally N-protected amino acid alkyl esters or optionally N-protected peptide alkyl esters with amino acid amides in an aqueous phase or aqueous-organic medium, in the presence of an enzyme which effects the peptide linkage and with enzymatic elimination of the amide protective group, in which process the synthesis is allowed to proceed continuously and the peptide amide is hydrolysed enzymatically by means of the peptide amidase to yield the peptide and, finally, the peptide is separated on the basis of its charge from the reaction mixture and the amino acid amide is recirculated.

**10.** Use of the microbial peptide amidase according to one of claims 3 to 5 for racemate resolution of N-protected amino acid amides, wherein a racemic mixture of N-protected amino acid amides is incubated with the peptide amidase and is reacted until complete conversion of the N-protectied L-amino acid amide and then the N-protected L-amino acid is separated from the N-protected D-amino acid amide on the basis of the difference in charge.

**11.** Use of the microbial peptide amidase according to one of claims 3 to 5 for obtaining non-proteogenic D-amino acids, wherein N-protected racemic amino acid amides such as N-acetylneopentylglycine amide, N-acetyl-naphthylalanine amide, N-acetylphenylglycine amide or similar derivatives are introduced, the N-acetyl-L-amino acid amides are hydrolysed enzymatically, the N-acetyl-D-amino acid amides are separated from the reaction mixture by chromatography and are finally converted into the free D-amino acids by acid hydrolysis.

**Revendications**

**1.** °) Procédé d'obtention de microorganismes contenant une peptidamidase par culture d'échantillons contenant des microorganismes dans des milieux nutritifs qui présentent une source de carbone, d'azote et de sels minéraux,
caractérisé en ce qu'
on fait tout d'abord incuber les échantillons dans un milieu nutritif présentant de l'azote d'amide comme source d'azote, puis en ce qu'on transinocule les colonies apparues dans un milieu nutritif qui contient du N-acétyl-D,L-méthioninamide, en ce qu'on fait incuber et en ce qu'on choisit les microorganismes qui croissent dans les deux milieux nutritifs puis qu'on en examine l'activité de peptidamidase.

**2.** °) Xanthomonas maltophilia déposée à la Deutsche Sammlung für Mikroorganismes sous le N° DSM 9181.

**3.** °) Peptidamidase microbienne apte à la séparation hydrolytique sélective du groupe amino libre à l'extrémité C-terminale des peptides amides,
caractérisé en ce qu'
on peut l'obtenir à partir d'un microorganisme produit selon la revendication 1.

**4.** °) Peptidamidase microbienne apte à la séparation hydrolytique sélective du groupe amino libre sur l'extrémité C-terminale des peptidamides, obtenus à partir des micoorganismes selon la revendication 2.

**5.** °) Peptidamidase microbienne selon la revendication 3 ou 4, après purification,
caractérisée par les paramètres suivants :

- séparation du groupe amino C-terminal des peptides amides et des amides d'acides aminés à protection N-terminale ;
- pas de coupure des liaisons peptidiques ;
- pH optimal situé à 6,0 ± 0,5 ;
- bonne stabilité dans un intervalle de pH compris entre 7 et 8 ;
- température optimale s'élevant à 35 à 40°C à un pH de 7,5 ;
- inhibition des radicaux sérine par des substances inhibitrices telles que le fluorure de phényl méthane sulfonyle

ainsi qu'en particulier le fluorure de 4-(2-aminoéthylbenzylsulfonyle) (Pefabloc) ;

- poids moléculaire s'élevant à environ 38 000 daltons (déterminé par filtration sur gel) ;
- point isoélectrique se situant à pH 5,8,
- une séquence de départ N-terminale suivante :

| | |
|---|---|
| AS 1 | X |
| AS 2 | Arg |
| AS 3 | Asn |
| AS 4 | Val |
| AS 5 | Pro |
| AS 6 | Phe |
| AS 7 | Pro |
| AS 8 | Tyr |
| AS 9 | Ala |
| AS 10 | Glu |
| AS 11 | Thr |
| AS 12 | Asp |
| AS 13 | Val |
| AS 14 | Ala |
| AS 15 | Asp |
| AS 16 | Leu |
| AS 17 | Gln |

et ses formes isozymes.

**6.** Utilisation de la peptidamidase microbienne selon l'une des revendications 3 à 5, dans un procédé de production de peptides et d'acides aminés protégés à l'extrémité N-terminale, de formule générale II

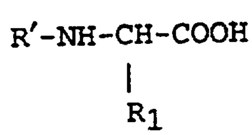

(II)

dans laquelle R' représente un groupe protecteur ou un radical acide aminé ou peptide quelconque lié par une liaison peptidique ou isopeptidique et $R_1$ représente un hydrogène ou une chaîne latérale quelconque, pour la séparation enzymatique d'un groupe amino C-terminal d'un peptidamide ou d'un amide d'acide aminé N-terminal protégé.

**7.** Utilisation selon la revendication 6,
caractérisé en ce qu'
on effectue la réaction enzymatique de façon continue.

**8.** Utilisation selon la revendication 6 ou 7,
caractérisée en ce qu'
on effectue la désamidation comme étape opératoire d'une réaction couplée avec un système enzymatique comprenant des protéases, des peptidases, des estérases et/ou des lipases.

**9.** Utilisation de la peptidamidase microbienne selon l'une des revendications 3 à 5 dans un procédé de production de peptides selon les revendications 6 à 8 par réaction enzymatique d'esters alkyliques d'acides aminés éventuellement N-protégés ou selon les cas d'esters alkyliques de peptides éventuellement N-protégés avec des amides d'acides aminés en phase aqueuse ou en milieu organique aqueux, en présence d'une enzyme produisant la liaison peptidique et avec séparation enzymatique du groupe protecteur d'amide, procédé dans lequel la synthèse se déroule de façon continue et le peptidamide est hydrolysé enzymatiquement au peptide au moyen de la peptidamidase, et enfin le peptide est séparé du mélange réactionnel de par sa charge et l'amide d'acide aminé est introduit.

**10.** Utilisation de la peptidamidase microbienne selon l'une des revendications 3 à 5 pour la séparation de racémates d'amides d'acides aminés N-protégés, dans laquelle on fait incuber un mélange racémique d'amides d'acides aminés N-protégés avec la peptidamidase et on fait réagir jusqu'à transformation complète de l'amide d'acide L-aminé N-protégé puis on sépare l'acide L-aminé N-protégé de l'acide D-aminé N-protégé sur la base des différences de charge.

**11.** Utilisation de la peptidamidase microbienne selon l'une des revendications 3 à 5 pour l'obtention d'acides D-aminé non protéinogène, dans laquelle on hydrolyse des amides d'acides aminés racémiques N-protégés comme le N-acétyl néopentylglycinamide, le N-acétylnaphtylalaninamide, le N-acétylphénylglycinamide ou des dérivés analogues, on hydrolyse enzymatiquement les amides d'acide N-acétyl-L-aminés, on sépare les amides d'acide N-acétyl-D-aminés par chromatographie du mélange réactionnel et enfin on les transforme en les acides D-aminés libres au moyen d'une hydrolyse acide.

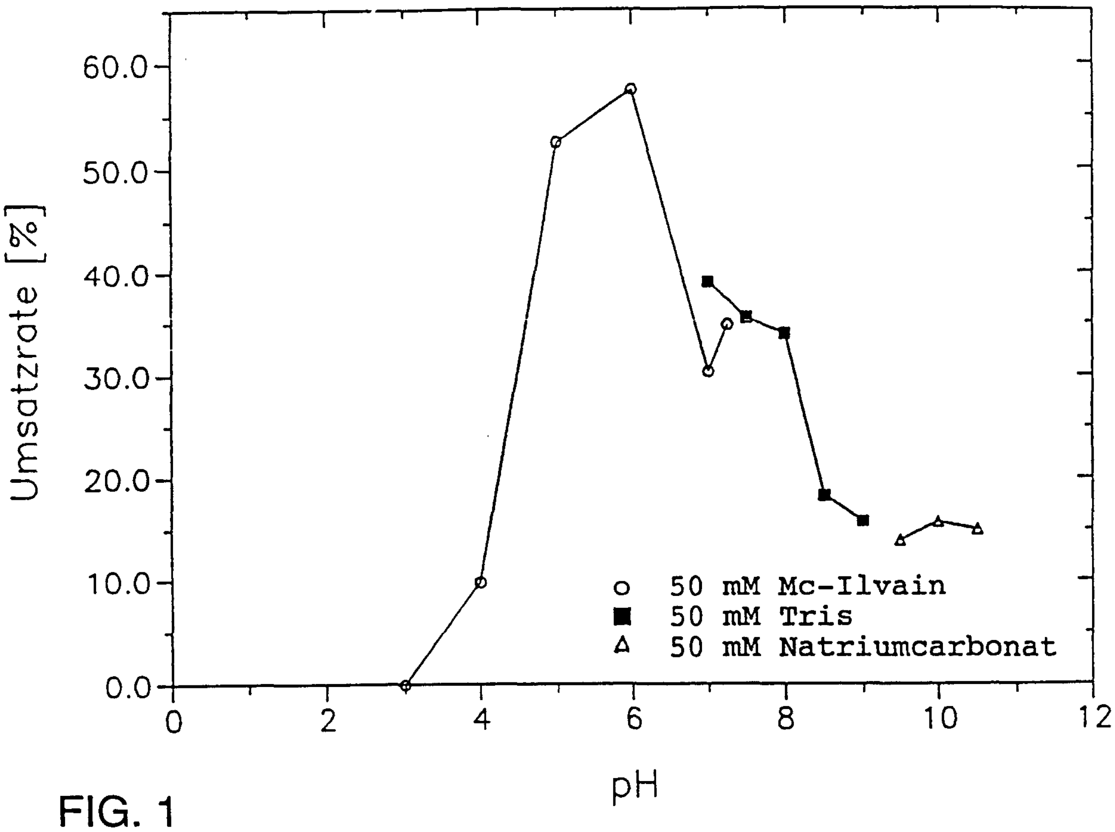

60.0
50.0
40.0
30.0
20.0
10.0
0.0
Umsatzrate [%]
0
2
4
6
8
10
12
pH
o 50 mM Mc-Ilvain
■ 50 mM Tris
Δ 50 mM Natriumcarbonat


FIG. 1

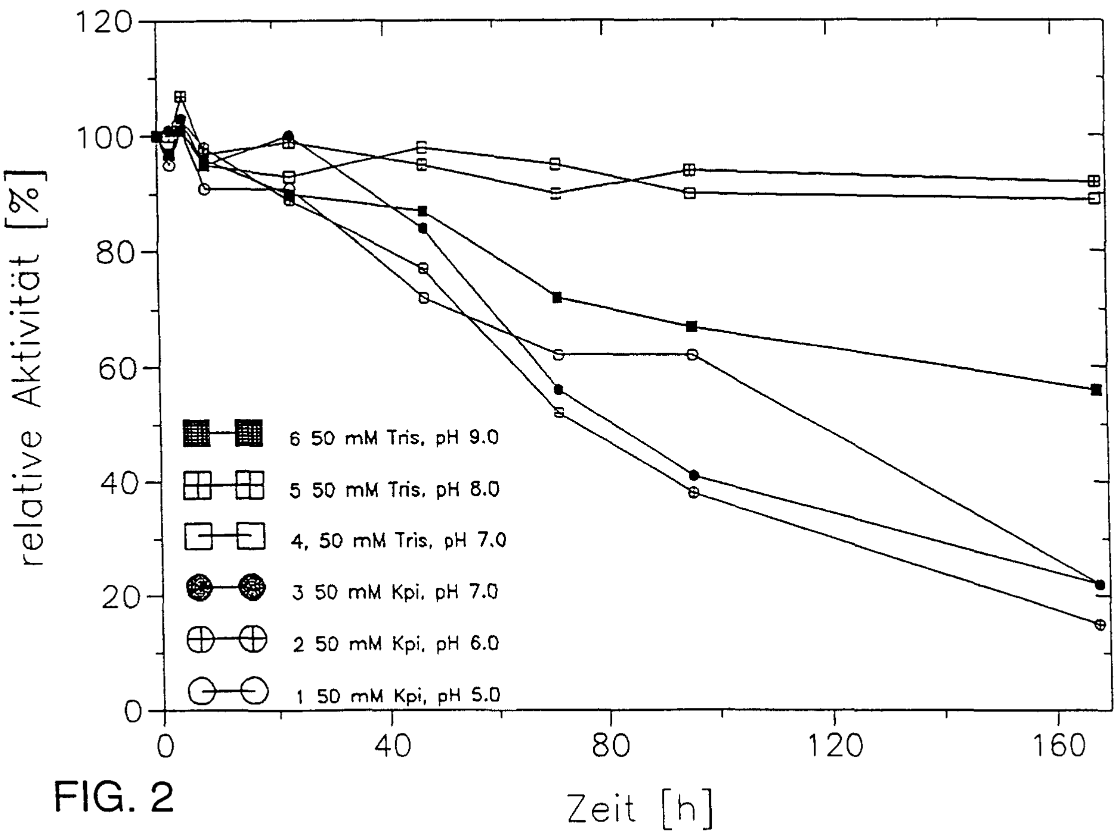

120
100
80
60
40
20
0
relative Aktivität [%]
6 50 mM Tris, pH 9.0
5 50 mM Tris, pH 8.0
4, 50 mM Tris, pH 7.0
3 50 mM Kpi, pH 7.0
2 50 mM Kpi, pH 6.0
1 50 mM Kpi, pH 5.0
0
40
80
120
160
Zeit [h]


FIG. 2

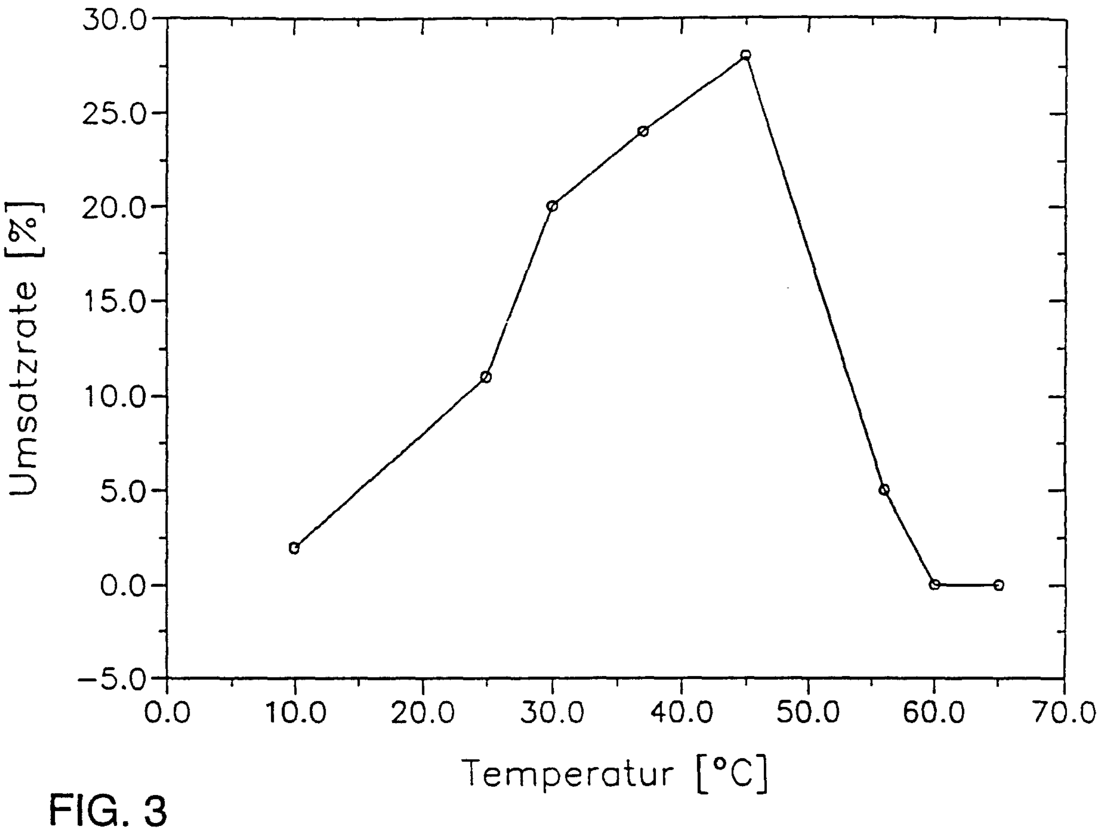
30.0
25.0
20.0
15.0
10.0
5.0
0.0
−5.0
Umsatzrate [%]
0.0
10.0
20.0
30.0
40.0
50.0
60.0
70.0
Temperatur [°C]

FIG. 3

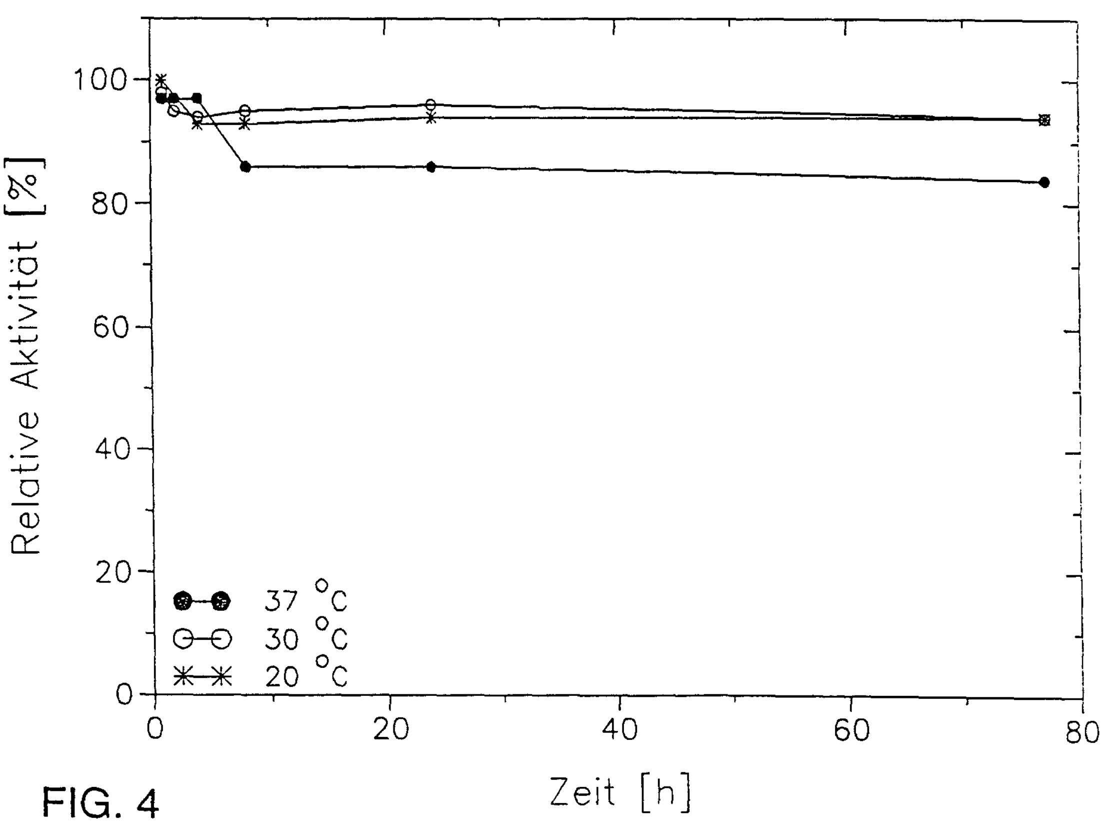
100
80
60
40
20
0
Relative Aktivität [%]
37 °C
30 °C
20 °C
0
20
40
60
80
Zeit [h]

FIG. 4

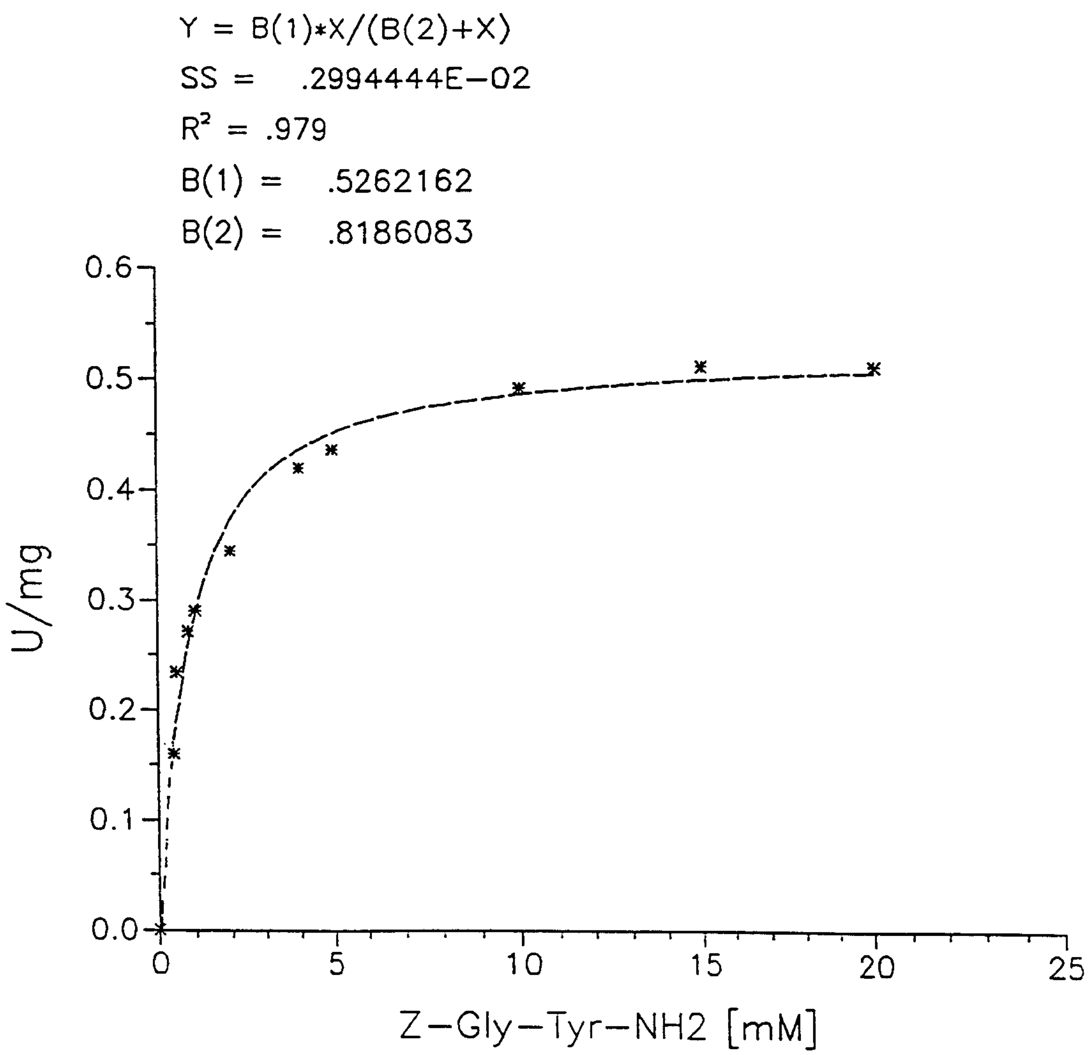
Y = B(1)*X/(B(2)+X)
SS = .2994444E-02
R² = .979
B(1) = .5262162
B(2) = .8186083
0.6
0.5
0.4
0.3
0.2
0.1
0.0
U/mg
0
5
10
15
20
25
Z-Gly-Tyr-NH2 [mM]

FIG. 5

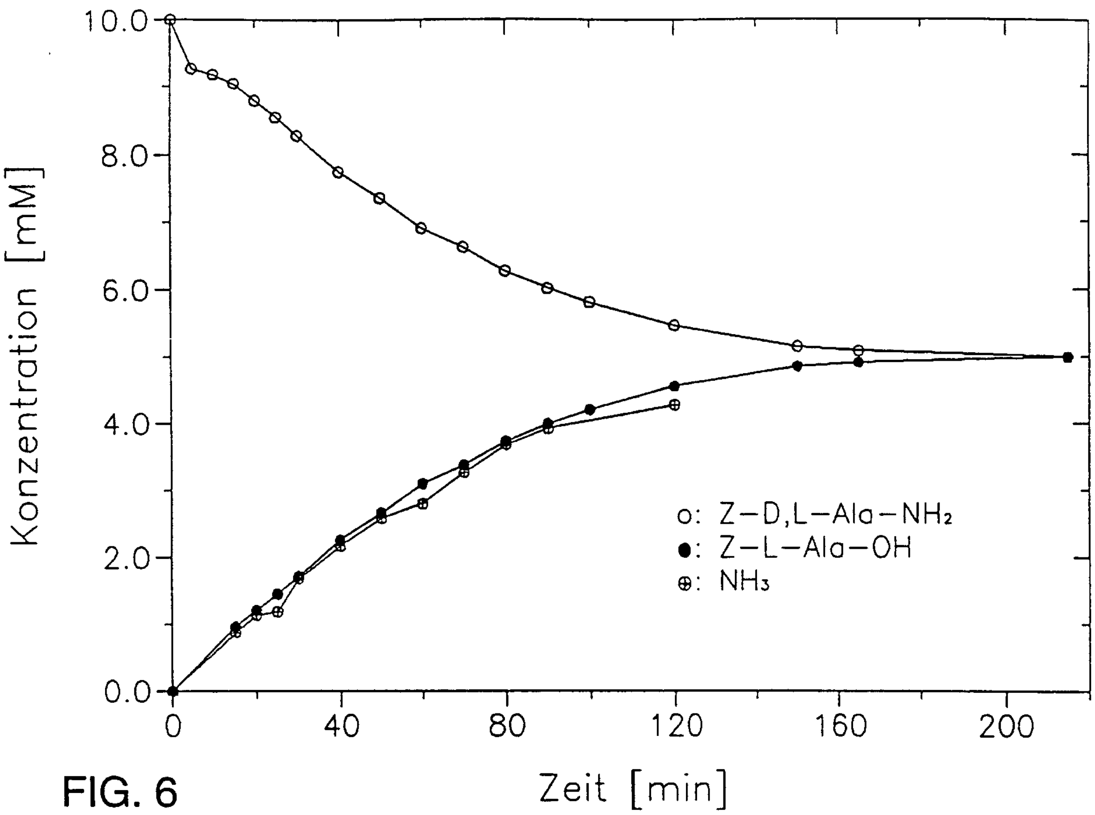
10.0
8.0
6.0
4.0
2.0
0.0
Konzentration [mM]
0
40
80
120
160
200
Zeit [min]
o: Z-D,L-Ala-NH₂
●: Z-L-Ala-OH
⊕: NH₃

FIG. 6

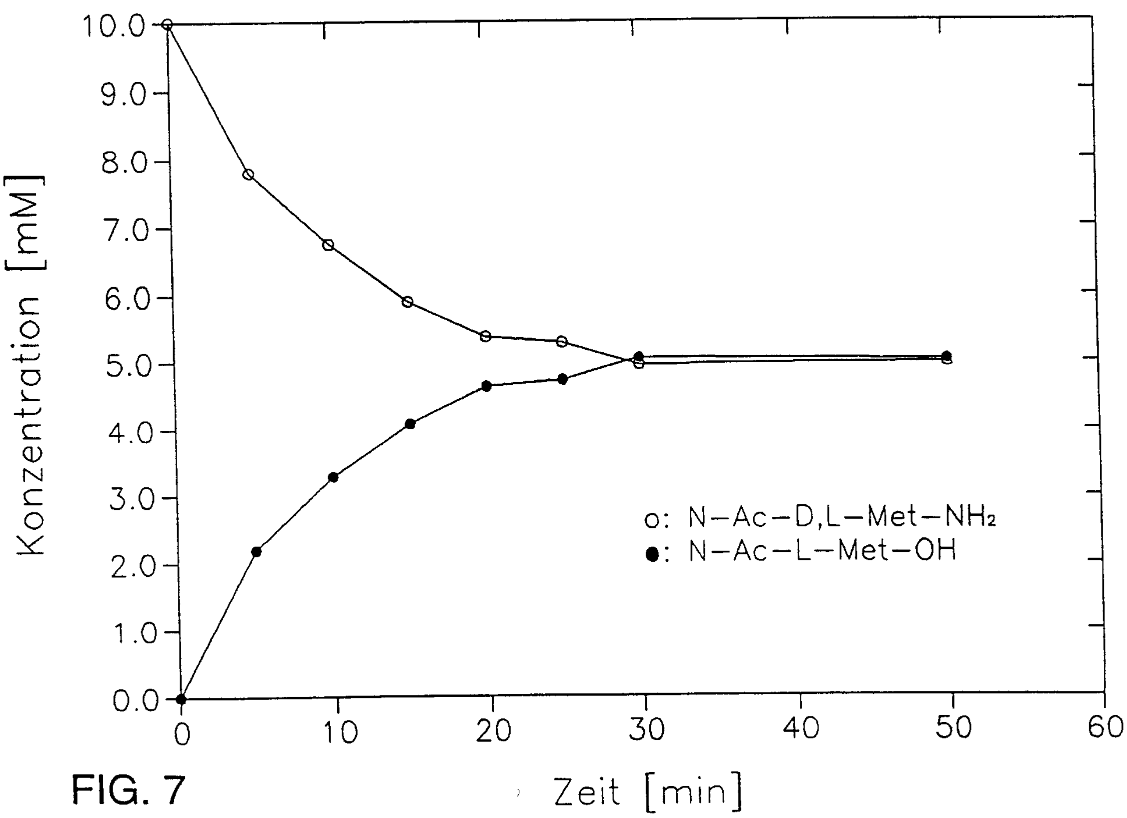
10.0
9.0
8.0
7.0
6.0
5.0
4.0
3.0
2.0
1.0
0.0
Konzentration [mM]
0
10
20
30
40
50
60
Zeit [min]
o: N-Ac-D,L-Met-NH₂
●: N-Ac-L-Met-OH

FIG. 7

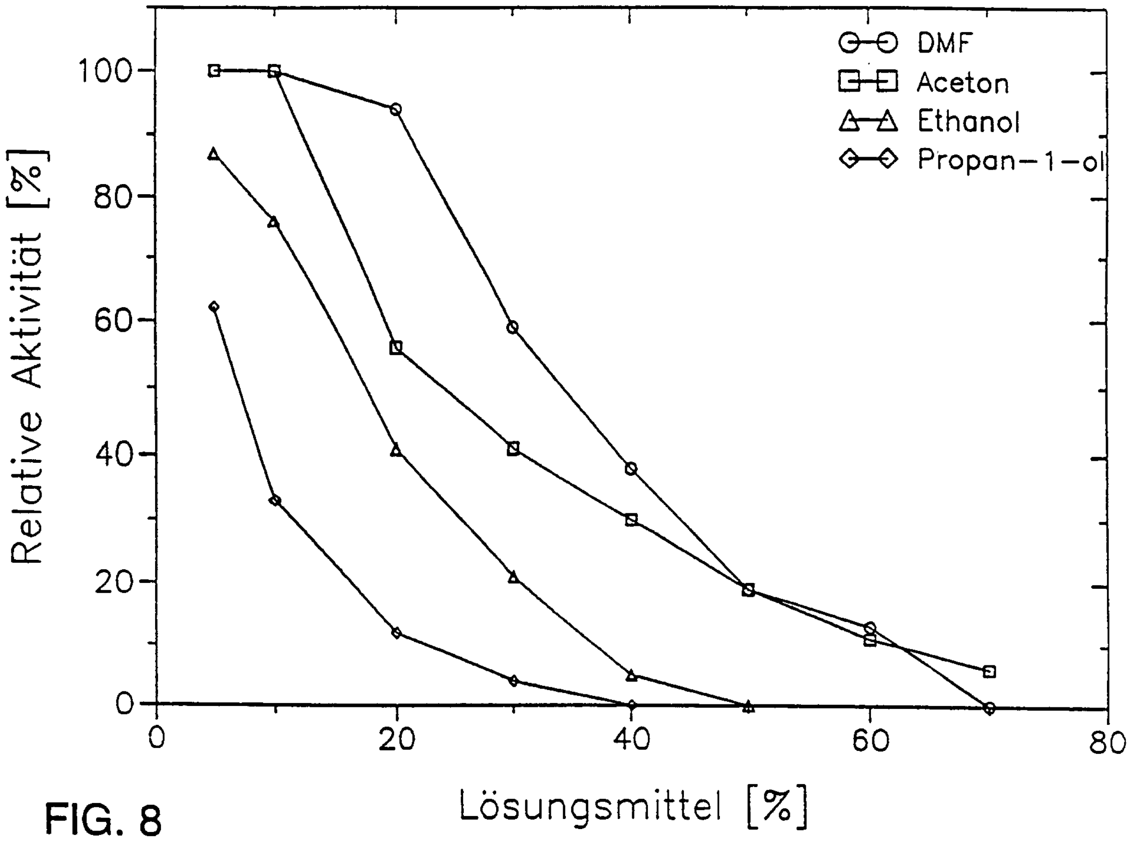
DMF
Aceton
Ethanol
Propan-1-ol
Relative Aktivität [%]
0
20
40
60
80
100
0
20
40
60
80
Lösungsmittel [%]

FIG. 8

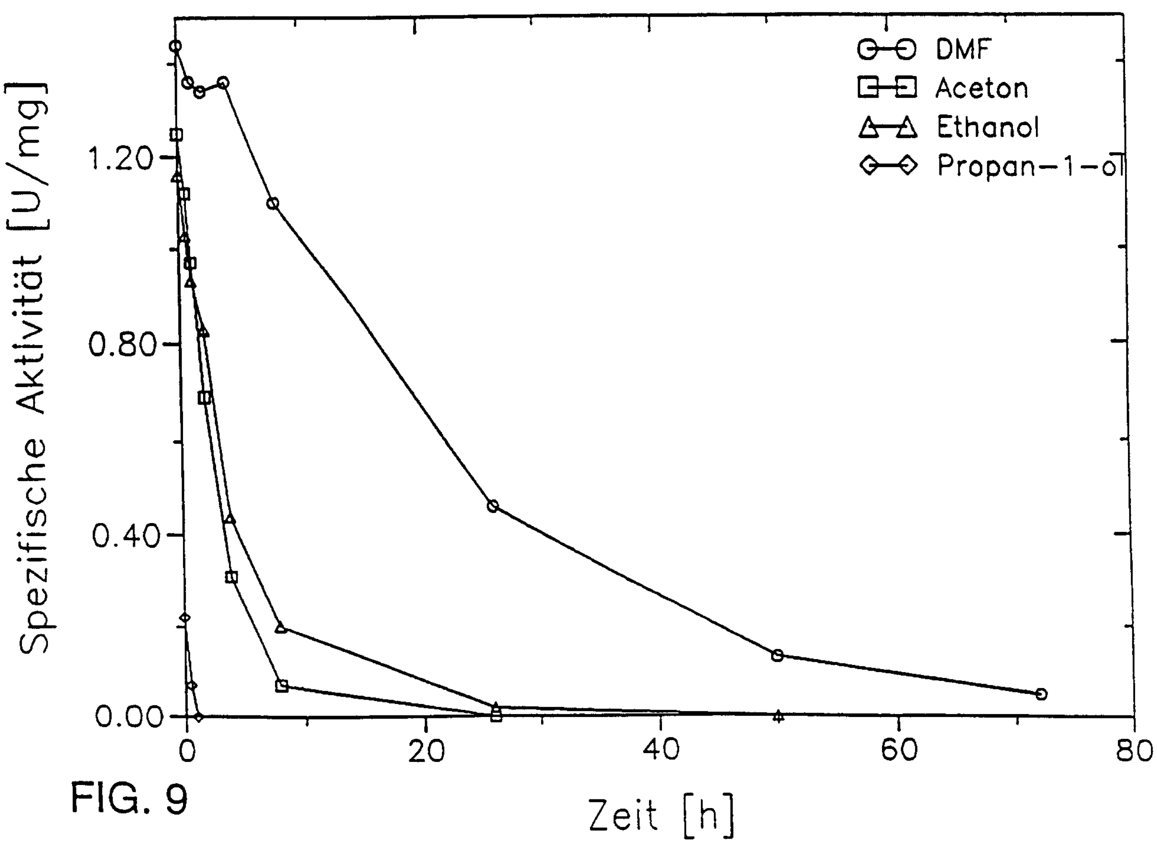
DMF
Aceton
Ethanol
Propan-1-ol
Spezifische Aktivität [U/mg]
0.00
0.40
0.80
1.20
0
20
40
60
80
Zeit [h]

FIG. 9